# EUROPEAN PATENT APPLICATION

(11) **EP 3 845 534 A1**
(43) Date of publication of application: **07.07.2021**
(21) Application number: 19855166.5
(22) Date of filing: 27.08.2019
(51) Int. Cl.: C07D 471/14, A61K 31/437, A61K 31/4375, A61K 31/4985, A61P 35/00

(54) **SALT FORM AND CRYSTAL FORM OF NOVEL AZATRICYCLIC COMPOUND AND USE THEREOF**

(30) Priority: 27.08.2018 WO PCT/CN2018/102549
(71) Applicant: Betta Pharmaceuticals Co., Ltd, Yuhang Hangzhou Zhejiang 311100 (CN)
(72) Inventor: XU, Xiaofeng, Beijing 100176 (CN); WANG, Jiabing, Beijing 100176 (CN); DING, Lieming, Hangzhou, Zhejiang 311100 (CN)
(74) Representative: Zwicker, Jörk
(86) International application number: PCT/CN2019/102720
(87) International publication number: WO 2020/043078

(57) **Abstract**

A maleate, mesylate, benzene sulfonate, hydrochloride, phosphate, L-tartrate, L-malate, citrate, and fumarate of a compound represented by structural formula I, various crystal forms of each salt form, and a preparation method and application thereof.

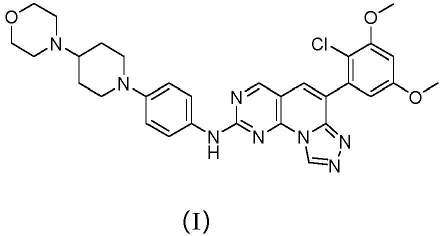

## Description

### FIELD OF THE INVENTION

The present invention relates to salt forms and crystalline forms of 6-(2-chloro-3,5-dimethoxyphenyl)-N-(4-(4-morpholinopiperidin-1-yl)phenyl)-[1,2,4]t riazolo[4',3':1,6]pyrido[2,3-d]pyrimidin-2-amine, methods of preparing the crystalline forms, pharmaceutical compositions comprising such crystalline forms, use of the new crystalline forms and the pharmaceutical compositions as FGFR inhibitor, and methods of using the new crystalline forms and the pharmaceutical compositions for treating FGFR-mediated diseases.

### BACKGROUND OF THE INVENTION

Protein kinases are enzymes that catalyze the phosphorylation of proteins, in most instances, the phosphorylation occurs on the serine (ser), threonine (thr) and tyrosine (tyr) residues of the protein. Many aspects of cell life processes (eg. cell growth, differentiation, proliferation, cell cycle and survival) are dependent on the activity of protein kinase. Furthermore, many diseases (eg. cancer and inflammation) are associated with the abnormal activity of protein kinase.

It has been found that Protein Tyrosine Kinase (PTK) have more than 100 family members so far, which play an important role in regulating cell differentiation, growth and proliferation. According to the structure of PTK, it can be divided into two types: receptor type PTK and non-receptor type PTK. The former is also known as transmembrane PTK, and the latter is also known as intracellular PTK.

Fibroblast growth factor receptors (FGFR) which belongs to a member of receptor tyrosine kinase (RTK) superfamily has become one of the targets for global pharmaceutical companies developing novel anti-tumor drugs. FGFR involves in the modulation of cell proliferation, apoptosis, migration, neovascularization and other processes. Due to its wide range of uses, FGFR and other RTK are regulated strictly in normal circumstances. In the tumor, such as breast cancer, bladder cancer, prostate cancer (currently developed indications), FGFR activation mutation or the over expression of ligand/receptor lead to its continuous activation, which is not only closely related to tumor occurrence, development, poor prognosis, but also plays a vital role in tumor angiogenesis, tumor invasion and metastasis. Therefore, FGFR is recognized as an important candidate for targeted cancer therapies, and the development of FGFR small molecular inhibitors has received more and more attention gradually.

The activation and transduction of fibroblast growth factor (FGFs): FGFs can initiate autophosphorylation of FGFRs on tyrosine residues of key activation loops in tyrosine kinase domain, which results in the transition of the tyrosine kinase domain from an inactive state to an activated state. (Bae J H, Schlessinger J. Molecules and Cells, 2010, 29(5): 443-448). The activated tyrosine kinase domain in FGFRs will phosphorylate other tyrosine residues progressively along the FGFRs-binding adaptor molecule at the substrate binding site. Phosphorylation of tyrosine residues in the C-terminal region of FGFRs enables phosphatase Cγ (PLCγ) to be absorbed and activated, Which catalyzes the conversion of phosphatidylinositol diphosphate (PIP2) to diglyceride (DAG) and triphosphate Alcohol (IP3). (Dailey L, Ambrostti D, Mansukhani A, et al. Cytokine & Growth Factor Reviews, 2005, 16(2), 233-247). Activated FGFR Phosphorylation Substrate 2 (FRS2) is capable of absorbing the Growth Factor Receptor Binding Protein 2 (GRB2) adaptor molecule.

FGFs signal can be transmitted to the Ras mitogen-activated protein kinase (Ras-MAPK) or PI3 kinase-protein kinase B (PI3K-AKT) signaling pathway via FRS2 and GRB2, and transmitted to protein kinase C (PKC) or protein kinase D (PKD) signaling pathway through PLCγ and DAG Kinase, and transmitted to the calcium ion release cascade pathway through PLCγ and IP3. FGFs-induced Ras-MAPK activation involves in cell proliferation, whereas FGFs-induced PI3K-AKT activation involves in cell survival.

FGFs signal participates in various aspects of tumor biology, such as anti-apoptosis, angiogenesis, Epithelial to Mesenchymal Transition (EMT), and invasion, etc. Targeted therapy of FGFRs has become the topical issues in the field of clinical oncology, and small molecule compounds designed and developed to fit the ATP binding POCket in the tyrosine kinase domain have been used in cancer therapy.

In human cancer cells with abnormal activation of FGFRs and anti-apoptotic potential, inhibition of FGFs signaling can reduce the load of cancer cells while inhibiting angiogenesis, and FGFR inhibitors can enhance the sensitivity of cancer cells against conventional anticancer drugs (such as 5-fluorouracil, irinotecan, paclitaxel, etc.). With the deepen understanding of FGFs signal networks and the intensive study of the mechanisms of action of FGFs and FGFRs, FGFR inhibitors with strong specificity and good therapeutic effects will be developed, therefore FGFR- targeted drugs may used to treat tumors will have extremely broad prospects.

Polymorphs of a particular organic pharmaceutical compound may have different physical properties, such as solubility, hygroscopicity and stability, due to their distinct three-dimensional crystal structures. However, it is generally not possible to predict whether a particular organic compound will form different crystalline forms, let alone predict the structure and properties of the crystalline forms themselves. Exploring new crystalline or polymorphic forms of a pharmaceutically useful compound provides a new opportunity for improving the overall characteristics of a pharmaceutical product and at the same time expands the variety of materials available to formulation scientists when designing. The discovery of new crystal forms of useful compounds has expanded the material variety for formulation design, which is obviously advantageous.

### BRIEF DESCRIPTION OF THE INVENTION

The present invention relates to the salt form of 6-(2-chloro-3,5-dimethoxyphenyl)-N-(4-(4-morpholinopiperidin-1-yl)phenyl)-[1,2,4]t riazolo[4',3':1,6]pyrido[2,3-d]pyrimidin-2-amine represented by Formula I, and the substantially pure crystalline form of each salt form.

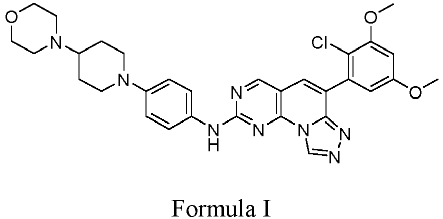

### The salt form of the compound of formula I

In some embodiments, one acid and compound I form a corresponding salt. Thes e salt-type compounds can exist in various physical forms. For example, it can be solutions, suspensions or solid forms. In some embodiments, the salt-type compound is in solid form. When in a solid form, the compound may be amorphous, crystalline or a mixture thereof. The salt forms formed by compound I and nine acids are exemplarily listed as follows. The nine acids are maleic acid, methanesulfonic acid, benzenesulfonic acid, hydrochloric acid, phosphoric acid, L-tartaric acid, L-malic acid, citric acid and fumaric acid.

### Exemplary examples of the crystalline forms of each salt form of the compound represented by structural formula I are as follows:

The present invention provides a preferable crystalline form of the maleate salt of compound of formula I, the X-ray powder diffraction pattern of this crystalline form comprises characteristic peaks with diffraction angles 2θ of 5.6°±0.2°, 8.4°±0.2°, 11.2°±0.2°, 22.6°±0.2° and 28.3°±0.2°. For convenience,it is called maleate salt crystalline form 1 in the present invention.

The present invention provides another preferable crystalline form of the maleate salt of compound represented by structural formula I, the X-ray powder diffraction pattern of this crystalline form comprises characteristic peaks with diffraction angles 2θ of 5.9°±0.2°. For convenience, it is called maleate salt crystalline form 2 in the present invention.

The present invention provides a preferable crystalline form of the methanesulfonate salt of compound represented by structural formula I, the X-ray powder diffraction pattern of this crystalline form comprises characteristic peaks with diffraction angles 2θ of 4.7°±0.2°, 9.4°±0.2° and 14.1°±0.2°. For convenience, it is called methanesulfonate salt crystalline form 1 in the present invention.

The present invention provides another preferable crystalline form of the methanesulfonate salt of compound represented by structural formula I, the X-ray powder diffraction pattern of this crystalline form comprises characteristic peaks with diffraction angles 2θ of 3.9°±0.2°, 15.7°±0.2° and 20.4°±0.2°. For convenience, it is called methanesulfonate salt crystalline form 2 in the present invention.

The present invention provides a preferable crystalline form of the benzenesulfonic acid (besylate)salt of compound represented by structural formula I, the X-ray powder diffraction pattern of this crystalline form comprises characteristic peaks with diffraction angles 2θ of 4.5°±0.2°,18.1°±0.2°,20.4°±0.2° and 21.5°±0.2°. For convenience, it is called besylate salt crystalline form 1 in the present invention.

The present invention provides another preferable crystalline form of the besylate salt of compound represented by structural formula I, the X-ray powder diffraction pattern of this crystalline form comprises characteristic peaks with diffraction angles 2θ of 4.0°±0.2°, 4.9°±0.2°, 6.9°±0.2° and 18.4°±0.2°. For convenience, it is called besylate salt crystalline form 2 in the present invention.

The present invention provides a preferable crystalline form of the hydrochloride salt of compound represented by structural formula I, the X-ray powder diffraction pattern of this crystalline form comprises characteristic peaks with diffraction angles 2θ of 4.2°±0.2°, 4.7°±0.2° and 6.9°±0.2°. For convenience, it is called hydrochloride salt crystalline form 1 in the present invention.

The present invention provides a preferable crystalline form of the phosphate salt of compound represented by structural formula I, the X-ray powder diffraction pattern of this crystalline form comprises characteristic peaks with diffraction angles 2θ of 7.5±0.2°, 15.1°±0.2°, 16.4±0.2°, 18.9±0.2° and 26.8±0.2°. For convenience, it is called phosphate salt crystalline form 1 in the present invention.

The present invention provides a preferable crystalline form of the L-tartrate salt of compound represented by structural formula I, the X-ray powder diffraction pattern of this crystalline form comprises characteristic peaks with diffraction angles 2θ of 7.6°±0.2°, 18.8°±0.2° and 27.3°±0.2°. For convenience, it is called L-tartrate salt crystalline form 1 in the present invention.

The present invention provides a preferable crystalline form of the L-malate salt of compound represented by structural formula I, the X-ray powder diffraction pattern of this crystalline form comprises characteristic peaks with diffraction angles 2θ of 3.6°±0.2°, 7.4°±0.2° and 18.8°±0.2°. For convenience, it is called L-malate salt crystalline form 1 in the present invention.

The present invention provides a preferable crystalline form of the citrate salt of compound represented by structural formula I, the X-ray powder diffraction pattern of this crystalline form comprises characteristic peaks with diffraction angles 2θ of 3.5°±0.2°, 7.3°±0.2° and 18.7°±0.2°. For convenience, it is called citrate salt crystalline form 1 in the present invention.

The present invention provides a preferable crystalline form of the fumarate salt of compound represented by structural formula I, the X-ray powder diffraction pattern of this crystalline form comprises characteristic peaks with diffraction angles 2θ of 5.5°±0.2°, 5.7°±0.2° and 19.3°±0.2°. For convenience, it is called fumarate salt crystalline form 1 in the present invention..

The present invention provides another preferable crystalline form of the fumarate salt of compound represented by structural formula I, the X-ray powder diffraction pattern of this crystalline form comprises characteristic peaks with diffraction angles 2θ of 4.5°±0.2°. For convenience, it is called fumarate salt crystalline form 2 in the present invention.

The X-ray powder diffraction patterns are all measured by Kalpha line of the Cu target.

The present invention further provides preferable embodiments of maleate salt crystalline form 1:
Preferably, the X-ray powder diffraction pattern of maleate salt crystalline form 1 comprises characteristic peaks with diffraction angles 2θ of 5.6°±0.2°, 8.4°±0.2°, 11.2°±0.2°, 17.0°±0.2°, 19.7±0.2°, 22.6°±0.2°, 25.4±0.2°, 28.3°±0.2°.

Preferably, the X-ray powder diffraction pattern of maleate salt crystalline form 1 comprises characteristic peaks with diffraction angles 2θ of 5.6°±0.2°, 8.4°±0.2°, 11.2°±0.2°, 17.0 ±0.2°, 19.7±0.2°, 22.6°±0.2°, 23.3°±0.2°, 25.4±0.2°, 28.3°±0.2°.

Preferably, the X-ray powder diffraction pattern of maleate salt crystalline form 1 comprises characteristic peaks with diffraction angles 2θ of 5.6°±0.2°, 8.4°±0.2°, 11.2°±0.2°, 14.0°±0.2°, 17.0°±0.2°, 19.7±0.2°, 22.6°±0.2°, 23.3°±0.2° , 25.4±0.2°, 28.3°±0.2°.

Preferably, the X-ray powder diffraction pattern of maleate salt crystalline form 1 comprises characteristic peaks with diffraction angles 2θ of 5.6°±0.2°, 8.4°±0.2°, 11.2°±0.2°, 15.3°±0.2°, 15.9°±0.2°, 17.0°±0.2°, 17.5°±0.2°, 18.5°±0.2°, 19.7°±0.2°, 22.6°±0.2°, 23.3°±0.2°, 25.4°±0.2°, 26.3°±0.2°, 27.5°±0.2° and 28.3°±0.2°.

More preferably, the main data of the X-ray powder diffraction pattern of maleate salt crystalline form 1 is shown in Table 1.

**Table 1**

| Number | 2θ± 0.2 (°) | Interplanar Spacing [Å] | Relative Intensity(%) |
|---|---|---|---|
| 1 | 5.6 | 15.77 | 50.9 |
| 2 | 8.4 | 10.54 | 46.9 |
| 3 | 11.2 | 7.86 | 47.5 |
| 4 | 14.0 | 6.31 | 14.9 |
| 5 | 15.3 | 5.77 | 93.1 |
| 6 | 15.6 | 5.66 | 36.3 |
| 7 | 15.9 | 5.56 | 75.9 |
| 8 | 16.3 | 5.42 | 39.1 |
| 9 | 17.0 | 5.20 | 48.2 |
| 10 | 17.5 | 5.05 | 83.4 |
| 11 | 17.9 | 4.95 | 17.9 |
| 12 | 18.3 | 4.85 | 22.9 |
| 13 | 18.5 | 4.79 | 43.0 |
| 14 | 19.7 | 4.50 | 80.5 |
| 15 | 20.1 | 4.41 | 25.2 |
| 16 | 21.9 | 4.05 | 11.3 |
| 17 | 22.6 | 3.94 | 93.2 |
| 18 | 23.3 | 3.82 | 40.9 |
| 19 | 25.0 | 3.56 | 10.1 |
| 20 | 25.4 | 3.50 | 63.1 |
| 21 | 26.3 | 3.39 | 48.2 |
| 22 | 27.5 | 3.25 | 53.4 |
| 23 | 28.3 | 3.15 | 100 |
| 24 | 30.4 | 2.94 | 11.9 |

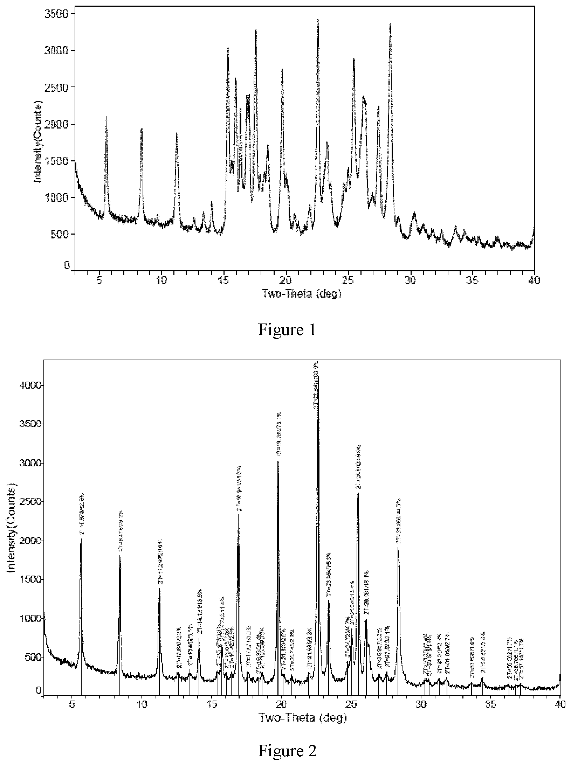

Preferably, the X-ray powder diffraction pattern of maleate salt crystalline form 1 is approximately as shown in Figure 1.

Preferably, the X-ray powder diffraction pattern of maleate salt crystalline form 1 is approximately as shown in Figure 2.

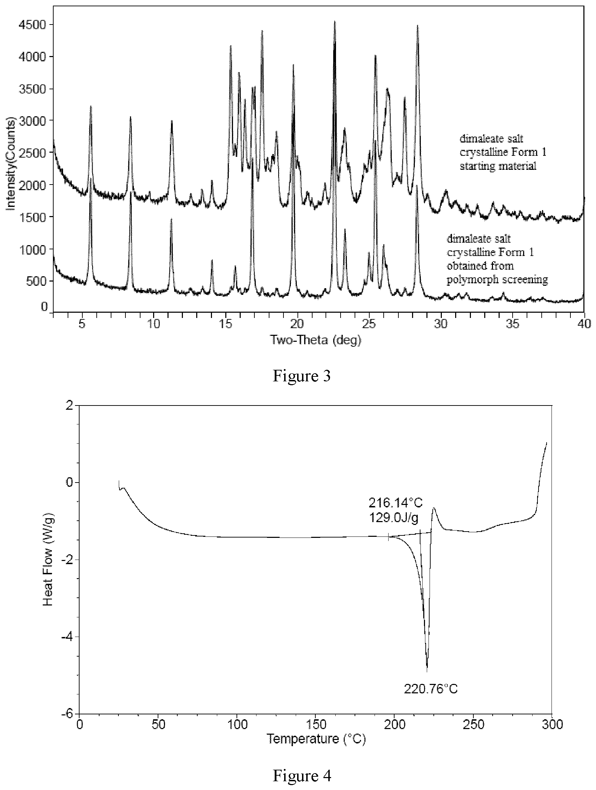

Further, maleate salt crystalline form 1 has a differential scanning calorimetry (DSC) thermogram substantially as shown in Figure 4. The onset temperature of endothermic peak of the maleate salt crystalline form 1 is about 216°C, and the peak temperature is about 221°C.

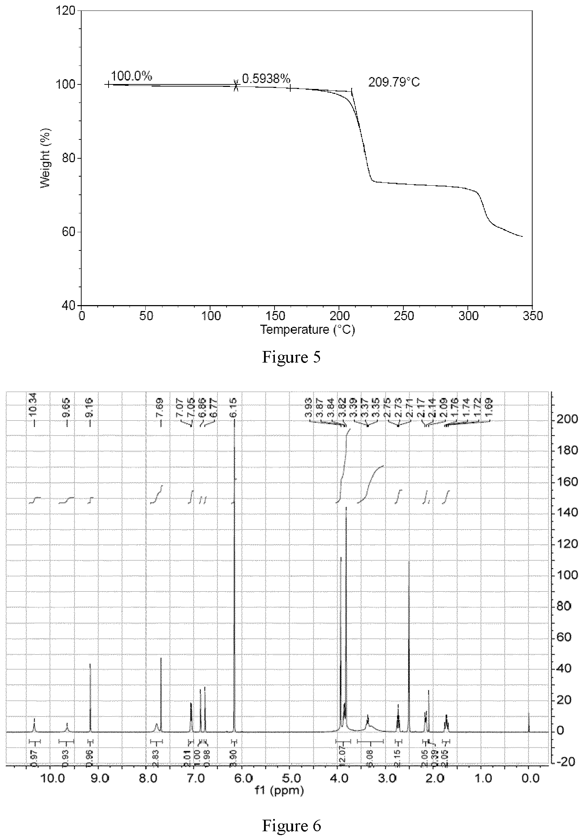

Further, the maleate salt crystalline form 1 has a thermal gravimetric analysis (TGA) thermogram substantially as shown in Figure 5. Further, maleate salt crystalline form 1 has a ¹H-NMR spectrum substantially as shown in Figure 6.

The present invention further provides preferable embodiments of maleate salt crystalline form 2:
Preferably, the X-ray powder diffraction pattern of maleate salt crystalline form 2 comprises characteristic peaks with diffraction angles 2θ of 5.9°±0.2°, 11.7°±0.2°, 17.6°±0.2°, 20.6°±0.2° and 23.5°±0.2°.

More preferably, the main data of the X-ray powder diffraction pattern of maleate salt crystalline form 2 is shown in Table 2.

**Table 2**

| Number | 2θ± 0.2 (°) | Interplanar Spacing [Å] | Relative Intensity(%) |
|---|---|---|---|
| 1 | 5.9 | 14.97 | 100 |
| 2 | 8.8 | 10.04 | 7.6 |
| 3 | 11.7 | 7.54 | 11.0 |
| 4 | 13.8 | 6.42 | 1.3 |
| 5 | 14.7 | 6.04 | 2.8 |
| 6 | 16.2 | 5.48 | 2.5 |
| 7 | 17.6 | 5.03 | 16.4 |
| 8 | 19.9 | 4.46 | 3.7 |
| 9 | 20.6 | 4.32 | 16.3 |
| 10 | 21.6 | 4.12 | 3.8 |
| 11 | 23.5 | 3.78 | 15.1 |
| 12 | 24.3 | 3.67 | 4.6 |
| 13 | 25.4 | 3.51 | 2.0 |
| 14 | 26.5 | 3.36 | 8.0 |
| 15 | 27.8 | 3.20 | 1.0 |
| 16 | 29.5 | 3.02 | 1.6 |
| 17 | 32.5 | 2.75 | 1.1 |
| 18 | 35.5 | 2.53 | 1.1 |
| 19 | 35.6 | 2.52 | 1.3 |
| 20 | 38.5 | 2.33 | 1.6 |

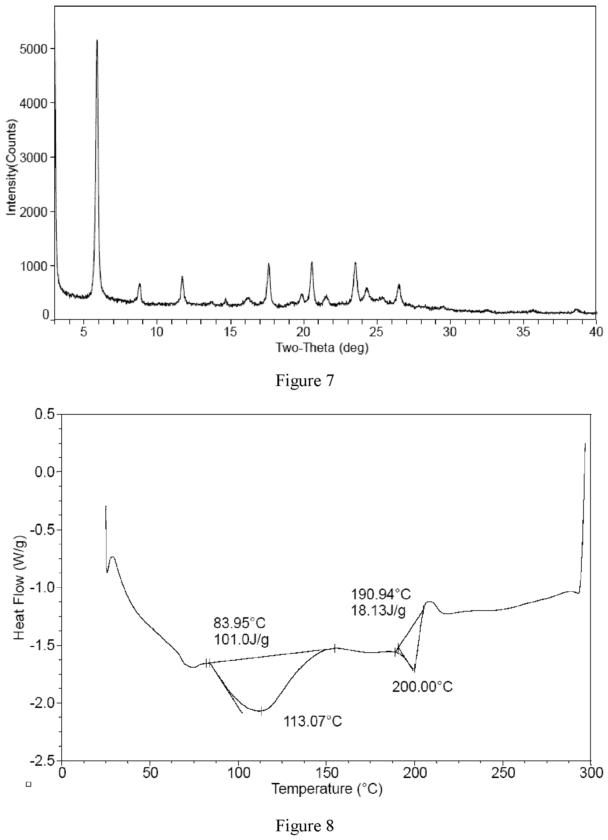

Preferably, the X-ray powder diffraction pattern of maleate salt crystalline form 2 is approximately as shown in Figure 7.

Further, maleate salt crystalline form 2 has a differential scanning calorimetry (DSC) thermogram substantially as shown in Figure 8.

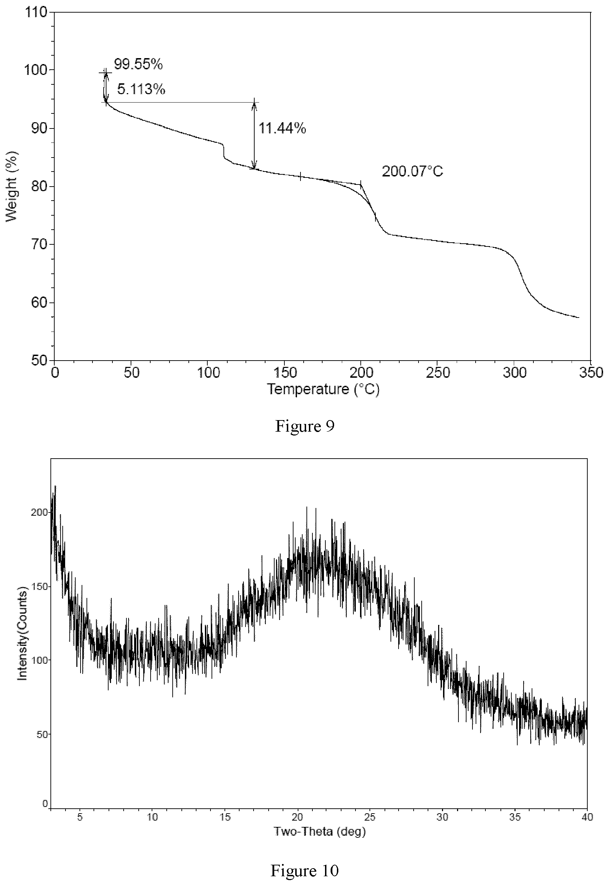

Further, maleate salt crystalline form 2 has a thermal gravimetric analysis (TGA) thermogram substantially as shown in Figure 9.

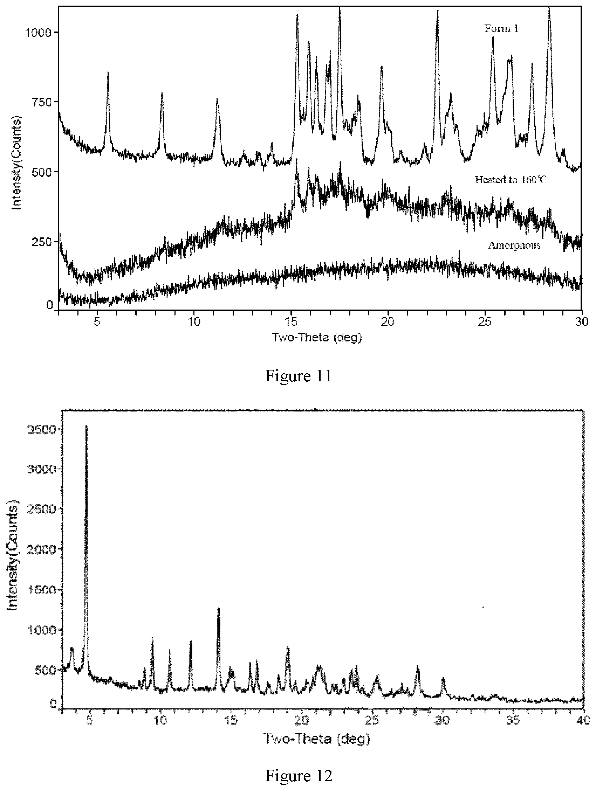

The present invention further provides an amorphous form of maleate salt of the compound of formula I,the amorphous form has an X-ray powder diffraction pattern shown in Figure 10, and it can be seen that no diffraction peak is seen. After the temperature is raised to 160°C, the diffraction peaks of the maleate salt crystalline form 1 are appeared, as shown in Figure 11.

The present invention further provides preferable embodiments of methanesulfonate salt crystalline form 1:
Preferably, the X-ray powder diffraction pattern of methanesulfonate salt crystalline form 1 comprises characteristic peaks with diffraction angles 2θ of 4.7°±0.2°, 9.4°±0.2°, 10.7°±0.2°, 12.1°±0.2°, 14.1°±0.2° and 19.0°±0.2°.

Preferably, the X-ray powder diffraction pattern of methanesulfonate salt crystalline form 1 comprises characteristic peaks with diffraction angles 2θ of 4.7°±0.2 °, 9.4°±0.2°, 10.7°±0.2°, 12.1°±0.2°, 14.1°±0.2°, 16.3°±0.2°, 16.8°±0.2° and 19.0°±0.2°.

Preferably, the X-ray powder diffraction pattern of methanesulfonate salt crystalline form 1 comprises characteristic peaks with diffraction angles 2θ of 4.7°±0.2 °, 9.4°±0.2°, 10.7°±0.2°, 12.1°±0.2°, 14.1°±0.2°, 16.3°±0.2°, 16.8°±0.2°, 19.0°±0.2°, 21.1°±0.2°, 21.4°±0.2°, 23.9°±0.2° and 28.2°±0.2°.

More preferably, the main data of the X-ray powder diffraction pattern of methanesulfonate salt crystalline form 1 is shown in Table 3.

**Table 3**

| Number | 2θ± 0.2 (°) | Interplanar Spacing [Å] | Relative Intensity(%) |
|---|---|---|---|
| 1 | 3.7 | 23.86 | 9.2 |
| 2 | 4.7 | 18.62 | 100 |
| 3 | 6.5 | 13.60 | 2.0 |
| 4 | 8.5 | 10.41 | 2.9 |
| 5 | 8.9 | 9.96 | 8.5 |
| 6 | 9.4 | 9.40 | 20.8 |
| 7 | 10.7 | 8.29 | 16.4 |
| 8 | 12.1 | 7.29 | 19.6 |
| 9 | 14.1 | 6.27 | 32.4 |
| 10 | 14.9 | 5.94 | 9.4 |
| 11 | 15.2 | 5.84 | 7.4 |
| 12 | 16.3 | 5.42 | 11.8 |
| 13 | 16.8 | 5.27 | 13.2 |
| 14 | 17.7 | 5.01 | 3.8 |
| 15 | 18.4 | 4.83 | 7.1 |
| 16 | 19.0 | 4.67 | 19.0 |
| 17 | 19.5 | 4.54 | 4.4 |
| 18 | 20.4 | 4.36 | 3.7 |
| 19 | 20.8 | 4.27 | 6.4 |
| 20 | 21.1 | 4.21 | 10.6 |
| 21 | 21.4 | 4.16 | 10.6 |
| 22 | 21.6 | 4.11 | 7.8 |
| 23 | 22.1 | 4.01 | 3.7 |
| 24 | 22.4 | 3.97 | 3.7 |
| 25 | 23.0 | 3.87 | 5.7 |
| 26 | 23.5 | 3.78 | 9.9 |
| 27 | 23.9 | 3.73 | 11.4 |
| 28 | 24.3 | 3.66 | 2.2 |
| 29 | 25.2 | 3.54 | 5.8 |
| 30 | 25.3 | 3.51 | 8.3 |
| 31 | 25.6 | 3.47 | 2.7 |
| 32 | 26.4 | 3.38 | 2.7 |
| 33 | 26.8 | 3.32 | 2.1 |
| 34 | 27.1 | 3.29 | 4.9 |
| 35 | 27.5 | 3.24 | 3.0 |
| 36 | 28.2 | 3.16 | 12.3 |
| 37 | 30.0 | 2.97 | 8.6 |
| 38 | 32.1 | 2.78 | 2.1 |
| 39 | 32.6 | 2.74 | 1.5 |
| 40 | 33.5 | 2.67 | 2.2 |
| 41 | 33.8 | 2.65 | 2.9 |
| 42 | 34.3 | 2.61 | 1.3 |
| 43 | 37.4 | 2.40 | 1.2 |
| 44 | 37.8 | 2.38 | 1.3 |
| 45 | 39.2 | 2.29 | 1.6 |

Preferably, the X-ray powder diffraction pattern of methanesulfonate salt crystalline form 1 is approximately as shown in Figure 12.

Futher, the solubility of methanesulfonate salt crystalline form 1 in water at 25°C is about 1.0mg/mL.

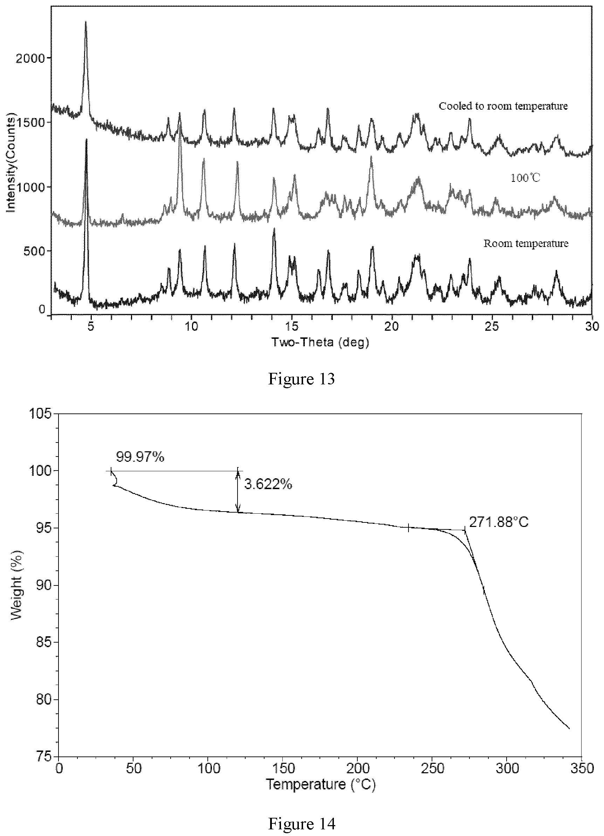

Futher, methanesulfonate salt crystalline form 1 has a hot-stage XRD pattern substantially as shown in Figure 13. Wherein, the crystal form has not changed when the methanesulfonate salt crystalline form 1 is heated to 100°C.

Further, methanesulfonate salt crystalline form 1 has a thermal gravimetric analysis (TGA) thermogram substantially as shown in Figure 14.

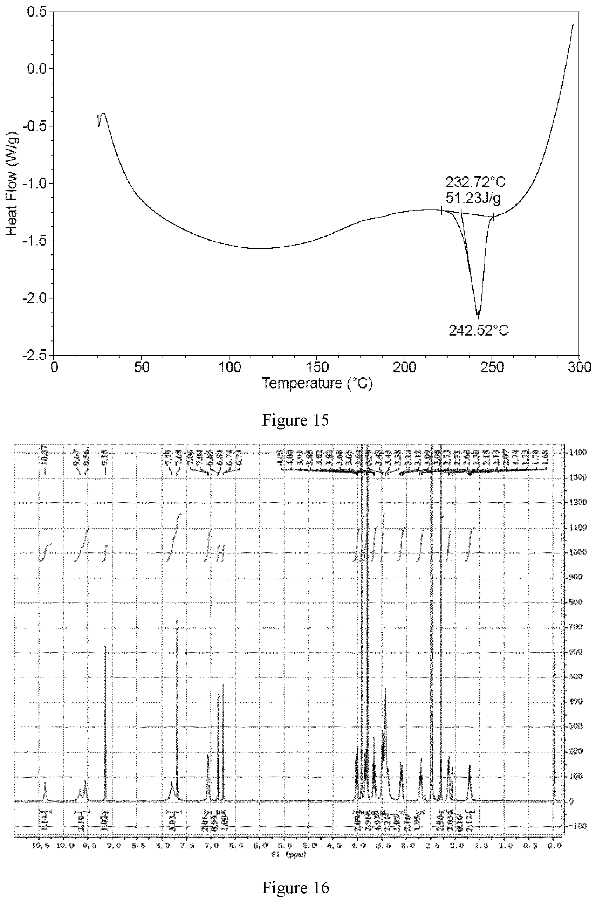

Further, methanesulfonate salt crystalline form 1 has a differential scanning calorimetry (DSC) thermogram substantially as shown in Figure 15.

Further, methanesulfonate salt crystalline form 1 has a ¹H-NMR spectrum substantially as shown in Figure 16.

The present invention further provides preferable embodiments of methanesulfonate salt crystalline form 2:
Preferably, the X-ray powder diffraction pattern of methanesulfonate salt crystalline form 2 comprises characteristic peaks with diffraction angles 2θ of 3.9°±0.2°, 4.8°±0.2°, 6.5°±0.2°, 13.0°±0.2°, 13.9°±0.2°, 15.7°±0.2° and 20.4°±0.2°.

Preferably, the X-ray powder diffraction pattern of methanesulfonate salt crystalline form 2 comprises characteristic peaks with diffraction angles 2θ of 3.9°±0.2°, 4.8°±0.2°, 6.5°±0.2°, 8.0°±0.2°, 11.9°±0.2°, 12.4°±0.2°, 13.0°±0.2°, 13.9°±0.2°, 15.7°±0.2°, 16.4°±0.2°, 18.7°±0.2°, 19.6°±0.2°, 19.8°±0.2°, 20.4°±0.2°, 21.6°±0.2°, 22.4°±0.2°, 23.7°±0.2° and 24.6 ±0.2°.

More preferably, the main data of the X-ray powder diffraction pattern of methanesulfonate salt crystalline form 2 is shown in Table 4.

**Table 4**

| Number | 2θ± 0.2 (°) | Interplanar Spacing [Å] | Relative Intensity(%) |
|---|---|---|---|
| 1 | 3.9 | 22.40 | 100 |
| 2 | 4.8 | 18.31 | 24.7 |
| 3 | 6.5 | 13.63 | 21.1 |
| 4 | 7.6 | 11.68 | 9.6 |
| 5 | 8.0 | 11.10 | 14.1 |
| 6 | 8.5 | 10.34 | 9.0 |
| 7 | 11.9 | 7.42 | 14.6 |
| 8 | 12.4 | 7.14 | 10.7 |
| 9 | 13.0 | 6.78 | 20.0 |
| 10 | 13.9 | 6.35 | 23.6 |
| 11 | 15.7 | 5.65 | 46.6 |
| 12 | 16.4 | 5.41 | 19.1 |
| 13 | 18.7 | 4.75 | 11.3 |
| 14 | 19.6 | 4.53 | 10.4 |
| 15 | 19.8 | 4.48 | 19.7 |
| 16 | 20.4 | 4.34 | 36.3 |
| 17 | 21.6 | 4.11 | 11.5 |
| 18 | 22.4 | 3.96 | 10.2 |
| 19 | 23.7 | 3.75 | 11.3 |
| 20 | 24.6 | 3.62 | 11.0 |
| 21 | 31.4 | 2.85 | 6.5 |

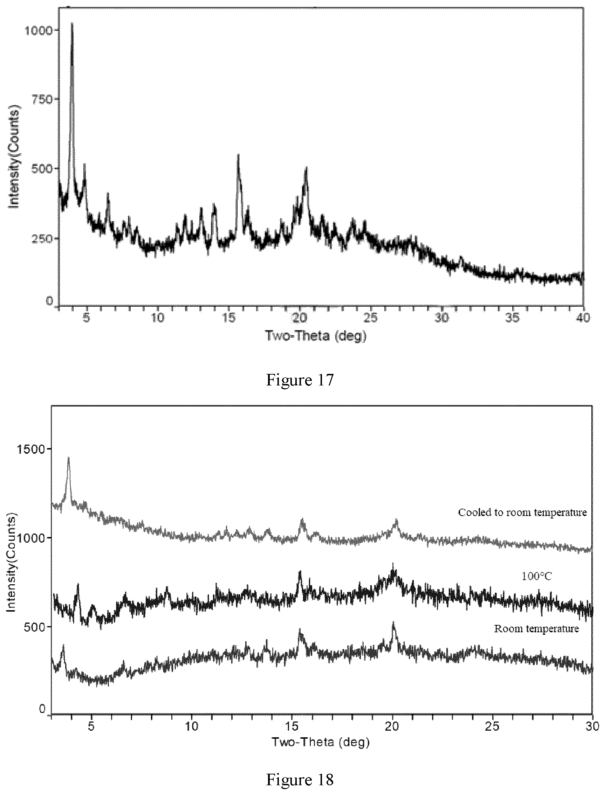

Preferably, the X-ray powder diffraction pattern of methanesulfonate salt crystalline form 2 is approximately as shown in Figure 17.

Futher, the solubility of the methanesulfonate salt crystalline form 2 in water at 25°C is about 5.0mg/mL.

Futher, methanesulfonate salt crystalline form 2 has a hot-stage XRD pattern substantially as shown in Figure 18. Wherein, the crystal form has not changed significantly when the methanesulfonate salt crystalline form 2 is heated to 100°C.

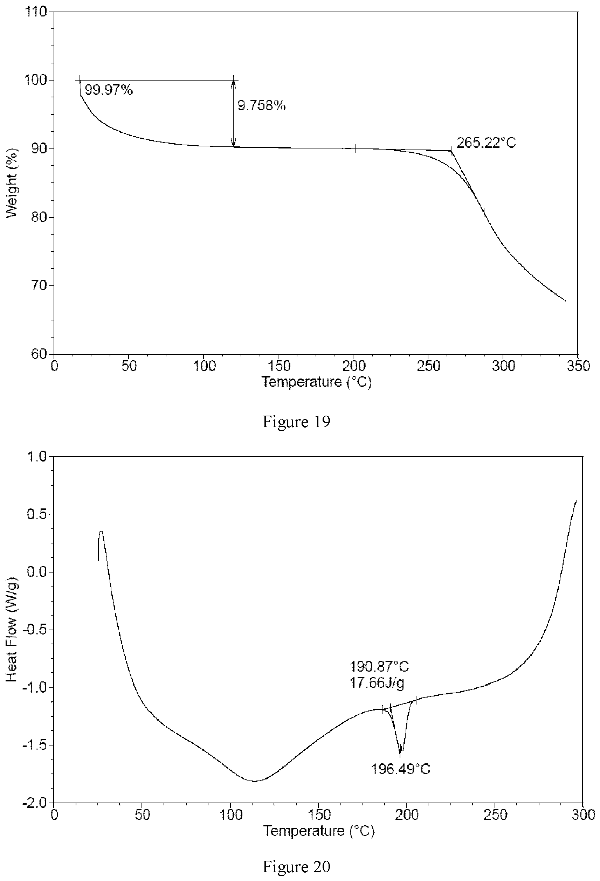

Further, methanesulfonate salt crystalline form 2 has a thermal gravimetric analysis (TGA) thermogram substantially as shown in Figure 19.

Further, methanesulfonate salt crystalline form 2 has a differential scanning calorimetry (DSC) thermogram substantially as shown in Figure 20.

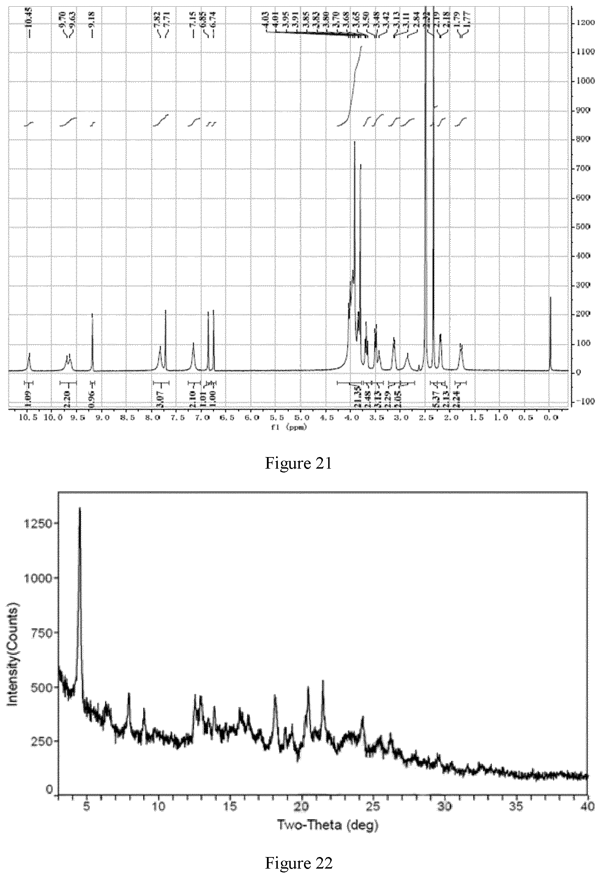

Further, methanesulfonate salt crystalline form 2 has a ¹H-NMR spectrum substantially as shown in Figure 21.

The present invention further provides preferable embodiments of besylate salt crystalline form 1:
Preferably, the X-ray powder diffraction pattern of besylate salt crystalline form 1 comprises characteristic peaks with diffraction angles 2θ of 4.5°±0.2°, 7.9°±0.2°, 12.5°±0.2°, 12.9°±0.2°, 18.1°±0.2°, 20.4°±0.2° and 21.5°±0.2°.

Preferably, the X-ray powder diffraction pattern of besylate salt crystalline form 1 comprises characteristic peaks with diffraction angles 2θ of 4.5°±0.2°, 6.3°±0.2°, 7.9°±0.2°, 9.0°±0.2°, 12.5°±0.2°, 12.9°±0.2°, 13.9°±0.2°, 15.7°±0.2°, 16.3°±0.2°, 18.1°±0.2°, 18.9°±0.2°, 19.3°±0.2°, 20.4°±0.2°, 21.5°±0.2°, 24.3°±0.2° and 26.2°±0.2°.

More preferably, the main data of the X-ray powder diffraction pattern of besylate salt crystalline form 1 is shown in Table 5.

**Table 5**

| Number | 2θ± 0.2 (°) | Interplanar Spacing [Å] | Relative Intensity(%) |
|---|---|---|---|
| 1 | 4.5 | 19.54 | 100 |
| 2 | 6.3 | 13.98 | 11.3 |
| 3 | 7.9 | 11.12 | 20.7 |
| 4 | 9.0 | 9.81 | 14.3 |
| 5 | 12.5 | 7.05 | 20.0 |
| 6 | 12.9 | 6.85 | 20.8 |
| 7 | 13.9 | 6.37 | 14.2 |
| 8 | 14.7 | 6.03 | 6.8 |
| 9 | 15.7 | 5.65 | 14.9 |
| 10 | 16.3 | 5.45 | 11.4 |
| 11 | 17.1 | 5.17 | 7.2 |
| 12 | 18.1 | 4.89 | 27.0 |
| 13 | 18.9 | 4.70 | 11.4 |
| 14 | 19.3 | 4.59 | 15.1 |
| 15 | 20.4 | 4.34 | 28.7 |
| 16 | 20.9 | 4.25 | 8.1 |
| 17 | 21.5 | 4.14 | 31.6 |
| 18 | 23.1 | 3.85 | 6.7 |
| 19 | 23.6 | 3.76 | 9.1 |
| 20 | 24.3 | 3.67 | 15.6 |
| 21 | 25.5 | 3.49 | 8.4 |
| 22 | 26.2 | 3.40 | 10.9 |
| 23 | 28.0 | 3.19 | 7.3 |
| 24 | 29.6 | 3.01 | 6.4 |
| 25 | 30.5 | 2.93 | 4.3 |
| 26 | 31.6 | 2.83 | 4.1 |
| 27 | 32.4 | 2.76 | 4.8 |

Preferably, the X-ray powder diffraction pattern of besylate salt crystalline form 1 is approximately as shown in Figure 22.

Futher, the solubility of the besylate salt crystalline form 1 in water at 25°C is about 0.08mg/mL.

Futher, besylate salt crystalline form 1 is analyzed by differential scanning calorimetry, the onset temperature of endothermic peak of besylate salt crystalline form 1 is about 224°C, and the peak temperature is about 234°C.

Futher, besylate salt crystalline form 1 is analyzed by thermal gravimetric analysis, it shows that besylate salt crystalline form 1 has a weight loss of about 3.8%before 120°C.

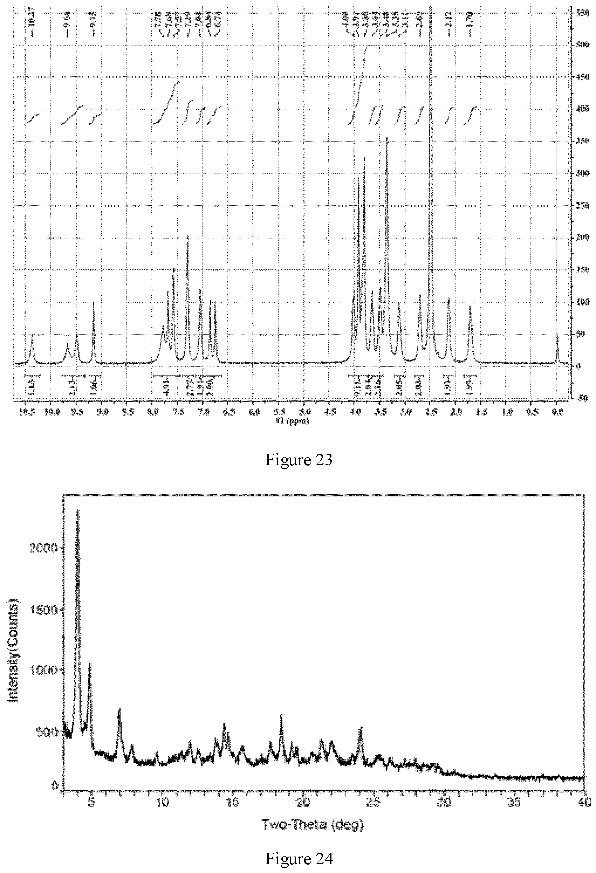

Further, besylate salt crystalline form 1 has a ¹H-NMR spectrum substantially as shown in Figure 23.

The present invention further provides preferable embodiments of besylate salt crystalline form 2:
Preferably, the X-ray powder diffraction pattern of besylate salt crystalline form 2 comprises characteristic peaks with diffraction angles 2θ of 4.0°±0.2°, 4.5°±0.2°, 4.9°±0.2°, 6.9°±0.2°, 14.4°±0.2°, 14.7°±0.2°, 18.4°±0.2° and 24.1°±0.2°.

More preferably, the main data of the X-ray powder diffraction pattern of besylate salt crystalline form 2 is shown in Table 6.

**Table 6**

| Number | 2θ± 0.2 (°) | Interplanar Spacing [Å] | Relative Intensity(%) |
|---|---|---|---|
| 1 | 4.0 | 22.18 | 100 |
| 2 | 4.5 | 19.80 | 10.4 |
| 3 | 4.9 | 18.16 | 34.1 |
| 4 | 6.9 | 12.73 | 22.2 |
| 5 | 7.9 | 11.21 | 7.6 |
| 6 | 9.6 | 9.21 | 5.5 |
| 7 | 11.4 | 7.75 | 4.5 |
| 8 | 12.0 | 7.38 | 8.4 |
| 9 | 12.5 | 7.05 | 5.9 |
| 10 | 13.8 | 6.43 | 8.2 |
| 11 | 14.4 | 6.15 | 15.9 |
| 12 | 14.7 | 6.04 | 10.6 |
| 13 | 15.7 | 5.64 | 7.1 |
| 14 | 17.0 | 5.20 | 3.6 |
| 15 | 17.7 | 5.01 | 7.5 |
| 16 | 18.4 | 4.81 | 20.1 |
| 17 | 19.2 | 4.62 | 8.9 |
| 18 | 19.5 | 4.55 | 7.0 |
| 19 | 20.6 | 4.30 | 3.5 |
| 20 | 21.3 | 4.17 | 9.3 |
| 21 | 21.9 | 4.05 | 8.9 |
| 22 | 22.3 | 3.98 | 4.1 |
| 23 | 23.4 | 3.79 | 4.2 |
| 24 | 24.1 | 3.70 | 16.2 |
| 25 | 25.5 | 3.50 | 5.0 |
| 26 | 26.2 | 3.39 | 4.2 |
| 27 | 27.2 | 3.28 | 3.6 |
| 28 | 27.9 | 3.19 | 3.5 |
| 29 | 29.2 | 3.06 | 3.7 |
| 30 | 37.0 | 2.43 | 2.2 |
| 31 | 38.1 | 2.36 | 2.2 |

Preferably, the X-ray powder diffraction pattern of besylate salt crystalline form 2 is approximately as shown in Figure 24.

Futher, the solubility of besylate salt crystalline form 2 in water at 25°C is about 1.0mg/mL.

Futher, the thermal gravimetric analysis shows that besylate salt crystalline form 2 has a weight loss of about 7.8%before 120°C.

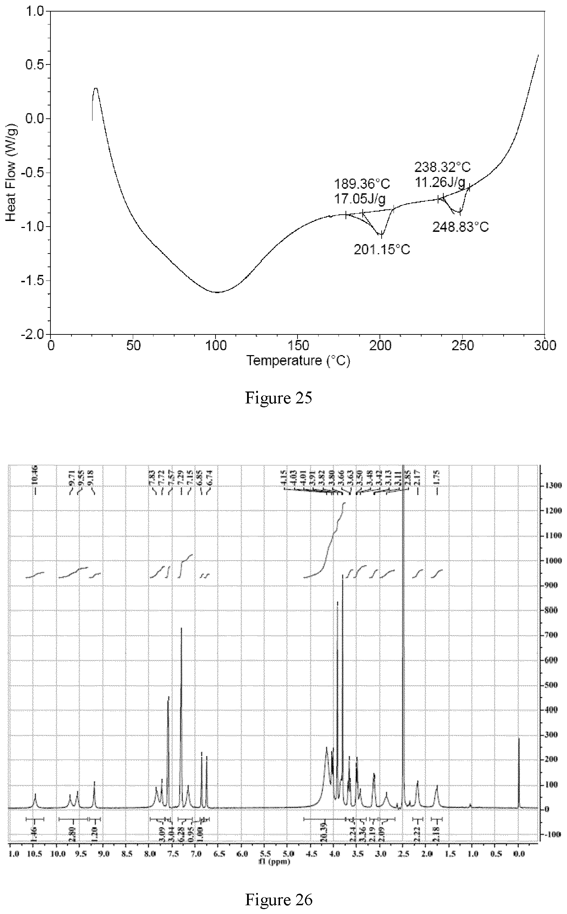

Further, besylate salt crystalline form 2 has a differential scanning calorimetry (DSC) thermogram substantially as shown in Figure 25.

Further, besylate salt crystalline form 2 has a ¹H-NMR spectrum as shown in Figure 26.

The present invention further provides preferable embodiments of hydrochloride salt crystalline form 1:
Preferably, the X-ray powder diffraction pattern of hydrochloride salt crystalline form 1 comprises characteristic peaks with diffraction angles 2θ of 4.2°±0.2°, 4.7°±0.2°, 6.9°±0.2°, 9.3°±0.2°, 12.1°±0.2°, 14.0°±0.2°, 14.6°±0.2°, 15.6°±0.2° and 19.7°±0.2°.

More preferably, the main data of the X-ray powder diffraction pattern of hydrochloride salt crystalline form 1 is shown in Table 7.

**Table 7**

| Number | 2θ± 0.2 (°) | Interplanar Spacing [Å] | Relative Intensity(%) |
|---|---|---|---|
| 1 | 4.2 | 21.21 | 100 |
| 2 | 4.7 | 18.80 | 24.6 |
| 3 | 6.9 | 12.83 | 36.1 |
| 4 | 7.8 | 11.31 | 2.1 |
| 5 | 8.3 | 10.59 | 2.7 |
| 6 | 9.3 | 9.48 | 17.2 |
| 7 | 10.5 | 8.39 | 2.5 |
| 8 | 12.1 | 7.31 | 16.9 |
| 9 | 12.4 | 7.15 | 5.7 |
| 10 | 13.7 | 6.46 | 4.3 |
| 11 | 14.0 | 6.33 | 14.7 |
| 12 | 14.6 | 6.08 | 10.8 |
| 13 | 15.6 | 5.67 | 11.2 |
| 14 | 16.7 | 5.31 | 9.8 |
| 15 | 18.6 | 4.78 | 4.0 |
| 16 | 19.7 | 4.51 | 14.0 |
| 17 | 20.9 | 4.25 | 4.1 |
| 18 | 21.3 | 4.17 | 3.3 |
| 19 | 22.6 | 3.93 | 3.8 |
| 20 | 23.3 | 3.81 | 7.2 |
| 21 | 23.7 | 3.75 | 2.4 |
| 22 | 24.3 | 3.66 | 4.6 |
| 23 | 24.7 | 3.60 | 5.9 |
| 24 | 25.2 | 3.53 | 4.1 |
| 25 | 27.3 | 3.26 | 5.3 |
| 26 | 28.1 | 3.18 | 4.3 |
| 27 | 29.0 | 3.07 | 6.6 |
| 28 | 31.2 | 2.86 | 3.4 |
| 29 | 35.4 | 2.53 | 1.9 |

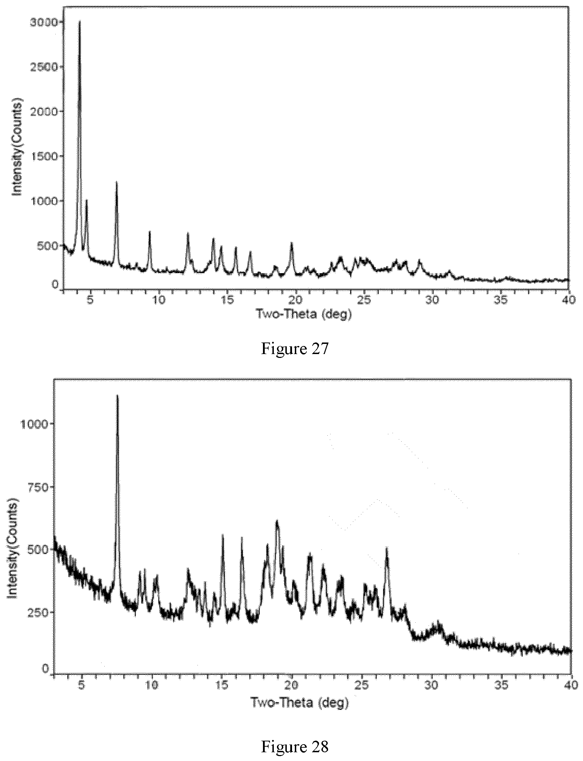

Preferably, the X-ray powder diffraction pattern of hydrochloride salt crystalline form 1 is approximately as shown in Figure 27.

Futher, the solubility of hydrochloride salt crystalline form 1 in water at 25°C is about 1.25mg/mL.

Futher, the differential scanning calorimetry analysis shows that the onset temperature of endothermic peak of the hydrochloride salt crystalline form 1 is about 216°C, and the peak temperature is about 223°C.

Futher, the thermal gravimetric analysis shows that hydrochloride salt crystalline form 1 has a weight loss of about 8.8% before 120°C.

The present invention further provides preferable embodiments of phosphate salt crystalline form 1:
Preferably, the X-ray powder diffraction pattern of phosphate salt crystalline form 1 comprises characteristic peaks with diffraction angles 2θ of 7.5°±0.2°, 12.6°±0.2°, 15.1°±0.2°, 16.4°±0.2°, 18.1°±0.2°, 18.3°±0.2°, 18.9°±0.2°, 19.4°±0.2°, 21.4°±0.2°, 22.2°±0.2° and 26.8°±0.2°.

Preferably, the X-ray powder diffraction pattern of phosphate salt crystalline form 1 comprises characteristic peaks with diffraction angles 2θ of 7.5°±0.2°, 9.2°±0.2°, 9.5°±0.2°, 10.4°±0.2°, 12.6°±0.2°, 13.0°±0.2°, 13.8°±0.2°, 14.5°±0.2°, 15.1°±0.2°, 16.4°±0.2°, 18.1°±0.2°, 18.3°±0.2°, 18.9°±0.2°, 19.4°±0.2°, 20.1°±0.2°, 21.4°±0.2°, 22.2°±0.2°, 23.3°±0.2°, 23.6°±0.2°, 25.3°±0.2°, 25.6°±0.2°, 26.0°±0.2°, 26.8°±0.2° and 28.1°±0.2°.

More preferably, the main data of the X-ray powder diffraction pattern of phosphate salt crystalline form 1 is shown in Table 8.

**Table 8**

| Number | 2θ± 0.2 (°) | Interplanar Spacing [Å] | Relative Intensity(%) |
|---|---|---|---|
| 1 | 7.5 | 11.74 | 100 |
| 2 | 9.2 | 9.65 | 18.0 |
| 3 | 9.5 | 9.30 | 19.2 |
| 4 | 10.4 | 8.52 | 17.4 |
| 5 | 12.6 | 7.01 | 21.2 |
| 6 | 13.0 | 6.78 | 13.8 |
| 7 | 13.4 | 6.61 | 8.1 |
| 8 | 13.8 | 6.41 | 15.9 |
| 9 | 14.5 | 6.11 | 11.8 |
| 10 | 15.1 | 5.87 | 39.3 |
| 11 | 16.4 | 5.39 | 38.8 |
| 12 | 18.1 | 4.91 | 20.1 |
| 13 | 18.3 | 4.85 | 26.2 |
| 14 | 18.9 | 4.68 | 35.8 |
| 15 | 19.4 | 4.58 | 24.8 |
| 16 | 20.1 | 4.41 | 12.1 |
| 17 | 20.3 | 4.36 | 9.2 |
| 18 | 21.4 | 4.15 | 25.6 |
| 19 | 22.2 | 4.00 | 22.2 |
| 20 | 23.3 | 3.82 | 15.4 |
| 21 | 23.6 | 3.77 | 17.5 |
| 22 | 24.4 | 3.65 | 7.3 |
| 23 | 25.3 | 3.52 | 15.2 |
| 24 | 25.6 | 3.48 | 12.7 |
| 25 | 26.0 | 3.43 | 13.5 |
| 26 | 26.8 | 3.33 | 32.9 |
| 27 | 28.1 | 3.18 | 10.3 |
| 28 | 30.0 | 2.98 | 5.3 |
| 29 | 34.4 | 2.60 | 5.1 |

Preferably, the X-ray powder diffraction pattern of phosphate salt crystalline form 1 is approximately as shown in Figure 28.

Futher, the solubility of phosphate salt crystalline form 1 in water at 25°C is about 0.1mg/mL.

Futher, the thermal gravimetric analysis shows that phosphate salt crystalline form 1 has a weight loss of about 4.0%before 120°C.

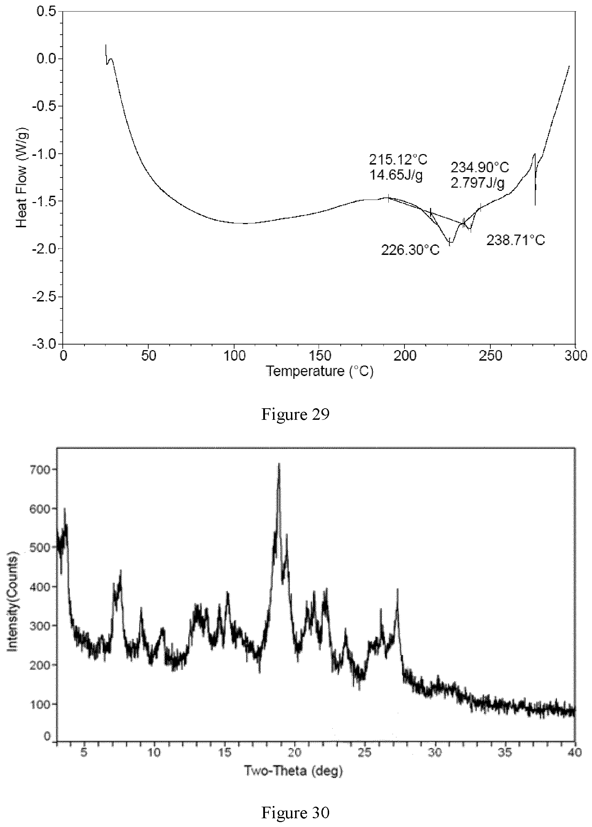

Further, phosphate salt crystalline form 1 has a differential scanning calorimetry (DSC) thermogram substantially as shown in Figure 29.

The present invention further provides preferable embodiments of L-tartrate salt crystalline form 1:
Preferably, the X-ray powder diffraction pattern of L-tartrate salt crystalline form 1 comprises characteristic peaks with diffraction angles 2θ of 3.6°±0.2°, 7.1°±0.2°, 7.6°±0.2°, 18.8°±0.2°, 19.4°±0.2° and 27.3 ±0.2°.

Preferably, the X-ray powder diffraction pattern of the L-tartrate salt crystalline form 1 comprises characteristic peaks with diffraction angles 2θ of 3.6°±0.2°, 7.1°±0.2°, 7.6°±0.2°, 9.0°±0.2°, 13.1°±0.2°, 14.6°±0.2°, 15.2°±0.2°, 18.8°±0.2°, 19.4°±0.2°, 20.9°±0.2°, 21.4°±0.2°, 22.1°±0.2°, 23.6°±0.2° and 27.3°±0.2°.

More preferably, the main data of the X-ray powder diffraction pattern of L-tartrate salt crystalline form 1 is shown in Table 9.

**Table 9**

| Number | 2θ± 0.2 (°) | Interplanar Spacing [Å] | Relative Intensity(%) |
|---|---|---|---|
| 1 | 3.6 | 24.63 | 35.9 |
| 2 | 7.1 | 12.47 | 38.2 |
| 3 | 7.6 | 11.65 | 46.6 |
| 4 | 9.0 | 9.77 | 27.5 |
| 5 | 10.6 | 8.34 | 18.0 |
| 6 | 13.1 | 6.76 | 26.6 |
| 7 | 13.7 | 6.45 | 14.5 |
| 8 | 14.6 | 6.06 | 21.1 |
| 9 | 15.2 | 5.84 | 27.3 |
| 10 | 18.8 | 4.71 | 100 |
| 11 | 19.4 | 4.57 | 36.4 |
| 12 | 20.9 | 4.24 | 20.2 |
| 13 | 21.4 | 4.15 | 24.3 |
| 14 | 22.1 | 4.02 | 28.6 |
| 15 | 23.6 | 3.77 | 24.1 |
| 16 | 25.4 | 3.50 | 13.0 |
| 17 | 26.3 | 3.39 | 15.9 |
| 18 | 27.3 | 3.26 | 44.1 |

Preferably, the X-ray powder diffraction pattern of L-tartrate salt crystalline form 1 is approximately as shown in Figure 30.

Futher, the solubility of L-tartrate salt crystalline form 1 in water at 25°C is about 0.08mg/mL.

Futher, the differential scanning calorimetry analysis shows the onset temperature of endothermic peak of the L-tartrate salt crystalline form 1 is about 201°C, and the peak temperature is about 213°C.

Futher, the thermal gravimetric analysis shows that L-tartrate salt crystalline form 1 has a weight loss of about 2.7%before 120°C.

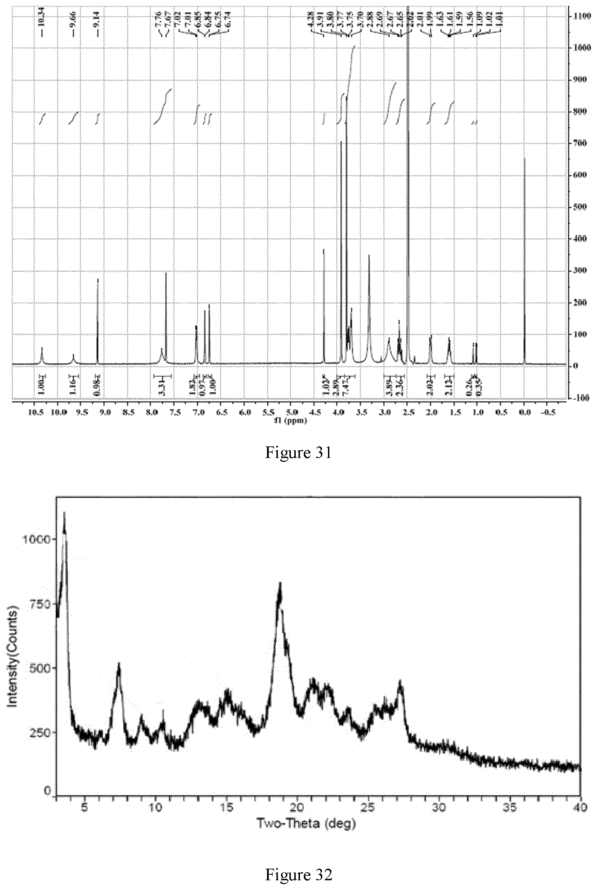

Further, L-tartrate salt crystalline form 1 has a ¹H-NMR spectrum substantially as shown in Figure 31.

The present invention further provides preferable embodiments of the L-malate salt crystalline form 1:
Preferably, the X-ray powder diffraction pattern of L-malate salt crystalline form 1 comprises characteristic peaks with diffraction angles 2θ of 3.6°±0.2°, 7.4°±0.2°, 18.8°±0.2° and 27.2°±0.2°.

Preferably, the X-ray powder diffraction pattern of L-malate salt crystalline form 1 comprises characteristic peaks with diffraction angles 2θ of 3.6°±0.2°, 7.4°±0.2°, 9.0°±0.2°, 10.5°±0.2°, 13.0°±0.2°, 15.1°±0.2°, 18.8°±0.2° and 27.2°±0.2°.

More preferably, the main data of the X-ray powder diffraction pattern of L-malate salt crystalline form 1 is shown in Table 10.

**Table 10**

| Number | 2θ± 0.2 (°) | Interplanar Spacing [Å] | Relative Intensity(%) |
|---|---|---|---|
| 1 | 3.6 | 24.85 | 100 |
| 2 | 7.4 | 11.93 | 36.2 |
| 3 | 9.0 | 9.79 | 12.7 |
| 4 | 10.5 | 8.40 | 14.7 |
| 5 | 13.0 | 6.80 | 13.2 |
| 6 | 13.7 | 6.48 | 8.2 |
| 7 | 14.5 | 6.09 | 6.0 |
| 8 | 15.1 | 5.85 | 12.2 |
| 9 | 16.0 | 5.52 | 7.2 |
| 10 | 18.8 | 4.72 | 58.2 |
| 11 | 21.3 | 4.17 | 8.6 |
| 12 | 22.2 | 4.00 | 9.7 |
| 13 | 23.6 | 3.76 | 8.8 |
| 14 | 25.5 | 3.48 | 7.9 |
| 15 | 26.1 | 3.41 | 8.4 |
| 16 | 27.2 | 3.27 | 20.4 |

Preferably, the X-ray powder diffraction pattern of L-malate salt crystalline form 1 is approximately as shown in Figure 32.

Futher, the solubility of L-malate salt crystalline form 1 in water at 25°C is about 0.25mg/mL.

Futher, the thermal gravimetric analysis shows that L-malate salt crystalline form 1 has a weight loss of about 3.3%.

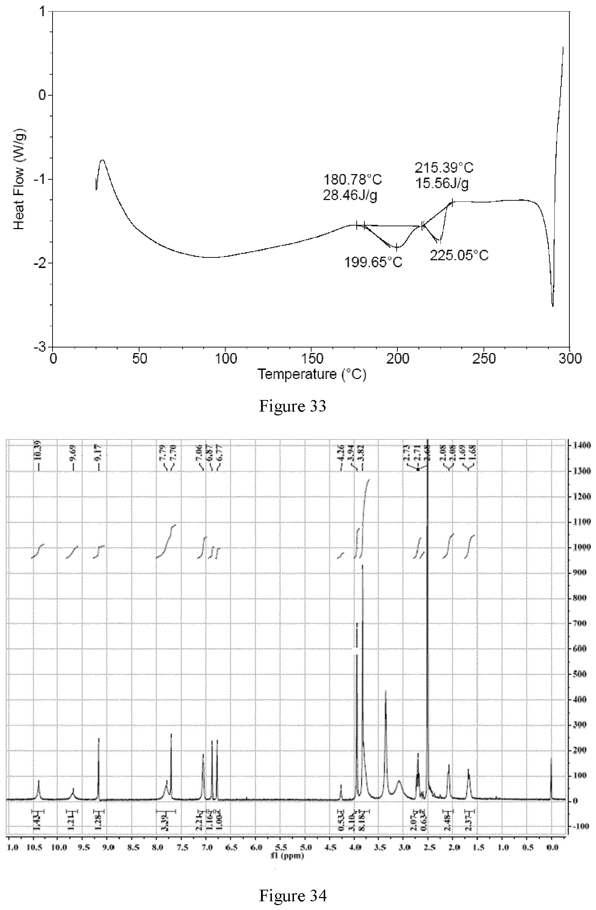

Further, L-malate salt crystalline form 1 has a differential scanning calorimetry (DSC) thermogram substantially as shown in Figure 33.

Further, L-malate salt crystalline form 1 has a ¹H-NMR spectrum substantially as shown in Figure 34.

The present invention further provides preferable embodiments of citrate salt crystalline form 1:
Preferably, the X-ray powder diffraction pattern of citrate salt crystalline form 1 comprises characteristic peaks with diffraction angles 2θ of 3.5°±0.2°, 7.3°±0.2°, 12.5°±0.2°, 14.9°±0.2°, 18.7°±0.2°, 21.0°±0.2° and 26.8°±0.2°.

More preferably, the main data of the X-ray powder diffraction pattern of citrate salt crystalline form 1 is shown in Table 11.

**Table 11**

| Number | 2θ± 0.2 (°) | Interplanar Spacing [Å] | Relative Intensity(%) |
|---|---|---|---|
| 1 | 3.5 | 25.13 | 100 |
| 2 | 6.1 | 14.51 | 5.1 |
| 3 | 7.3 | 12.12 | 35.4 |
| 4 | 9.0 | 9.83 | 7.0 |
| 5 | 10.2 | 8.71 | 4.2 |
| 6 | 12.5 | 7.05 | 12.1 |
| 7 | 13.2 | 6.72 | 4.8 |
| 8 | 14.9 | 5.94 | 10.4 |
| 9 | 15.5 | 5.73 | 4.7 |
| 10 | 16.1 | 5.51 | 7.1 |
| 11 | 18.7 | 4.75 | 31.9 |
| 12 | 21.0 | 4.22 | 10.5 |
| 13 | 22.1 | 4.01 | 7.2 |
| 14 | 23.4 | 3.80 | 5.3 |
| 15 | 25.4 | 3.50 | 2.8 |
| 16 | 26.8 | 3.32 | 10.7 |
| 17 | 30.1 | 2.97 | 4.5 |
| 18 | 38.1 | 2.36 | 2.2 |

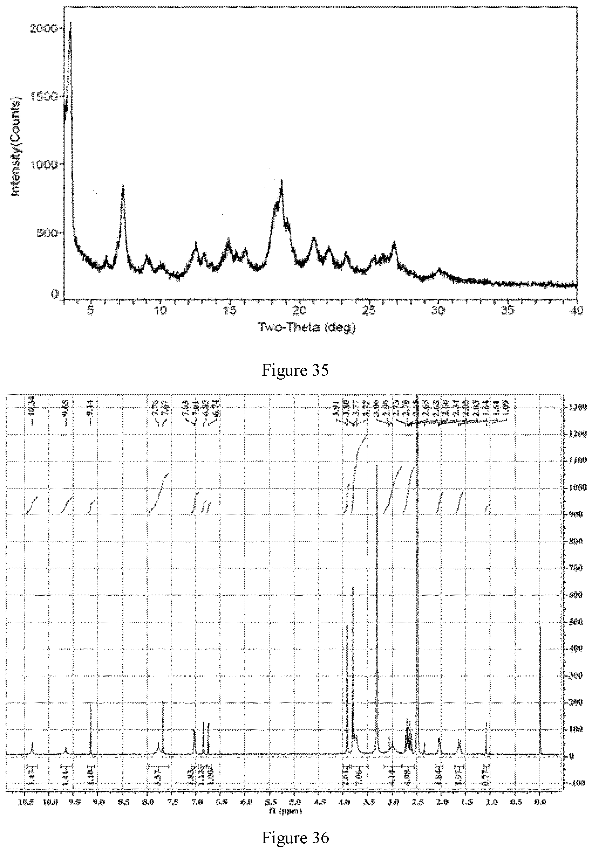

Preferably, the X-ray powder diffraction pattern of citrate salt crystalline form 1 is approximately as shown in Figure 35.

Futher, the solubility of citrate salt crystalline form 1 in water at 25°C is about 0.08mg/mL.

Futher, the differential scanning calorimetry analysis shows the onset temperature of endothermic peak of the citrate salt crystalline form 1 is about 164°C, and the peak temperature is about 188°C.

Futher, the thermal gravimetric analysis shows that citrate salt crystalline form 1 has a weight loss of about 2.2%before 120°C.

Further, citrate salt crystalline form 1 has a ¹H-NMR spectrum substantially as shown in Figure 36.

The present invention further provides preferable embodiments of fumarate salt crystalline form 1:
Preferably, the X-ray powder diffraction pattern of fumarate salt crystalline form 1 comprises characteristic peaks with diffraction angles 2θ of 5.5°±0.2°, 5.7°±0.2°, 17.0°±0.2°, 19.3°±0.2° and 22.2°±0.2°.

Preferably, the X-ray powder diffraction pattern of fumarate salt crystalline form 1 comprises characteristic peaks with diffraction angles 2θ of 5.5°±0.2°, 5.7°±0.2°, 17.0°±0.2°, 19.3°±0.2°, 22.2°±0.2°, 23.5°±0.2° and 27.0°±0.2°.

Preferably, the X-ray powder diffraction pattern of fumarate salt crystalline form 1 comprises characteristic peaks with diffraction angles 2θ of 5.5°±0.2°, 5.7°±0.2°, 10.8°±0.2°, 13.6°±0.2°, 16.1°±0.2°, 17.0°±0.2°, 18.2°±0.2°, 19.3°±0.2°, 20.3°±0.2°, 22.2°±0.2°, 23.5°±0.2°, 24.9°±0.2° and 27.0°±0.2°.

More preferably, the main data of the X-ray powder diffraction pattern of fumarate salt crystalline form 1 is shown in Table 12.

**Table 12**

| Number | 2θ± 0.2 (°) | Interplanar Spacing [Å] | Relative Intensity(%) |
|---|---|---|---|
| 1 | 5.5 | 16.17 | 54.6 |
| 2 | 5.7 | 15.44 | 100 |
| 3 | 9.0 | 9.86 | 7.1 |
| 4 | 9.5 | 9.31 | 5.9 |
| 5 | 10.5 | 8.43 | 6.5 |
| 6 | 10.8 | 8.18 | 12.9 |
| 7 | 13.0 | 6.81 | 7.4 |
| 8 | 13.6 | 6.49 | 10.2 |
| 9 | 15.4 | 5.75 | 3.3 |
| 10 | 15.8 | 5.62 | 6.9 |
| 11 | 16.1 | 5.49 | 16.5 |
| 12 | 17.0 | 5.20 | 32.0 |
| 13 | 18.2 | 4.86 | 12.2 |
| 14 | 19.3 | 4.59 | 57.8 |
| 15 | 20.3 | 4.38 | 11.3 |
| 16 | 21.6 | 4.10 | 7.4 |
| 17 | 22.2 | 4.01 | 31.2 |
| 18 | 22.7 | 3.92 | 8.0 |
| 19 | 23.5 | 3.78 | 20.7 |
| 20 | 24.9 | 3.58 | 11.7 |
| 21 | 27.0 | 3.30 | 20.4 |
| 22 | 28.4 | 3.14 | 4.7 |
| 23 | 31.3 | 2.85 | 4.1 |
| 24 | 34.1 | 2.63 | 2.8 |

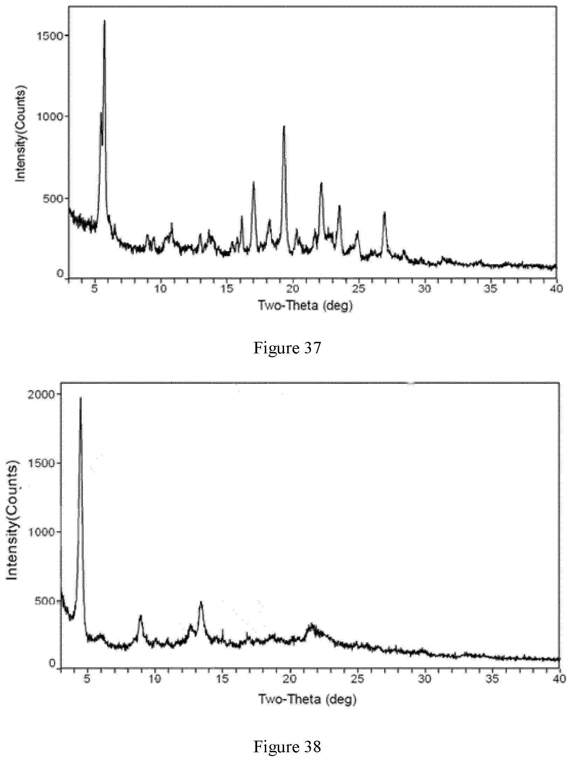

Preferably, the X-ray powder diffraction pattern of fumarate salt crystalline form 1 is approximately as shown in Figure 37.

The present invention further provides preferable embodiments of fumarate salt crystalline form 2:
Preferably, the X-ray powder diffraction pattern of fumarate salt crystalline form 2 comprises characteristic peaks with diffraction angles 2θ of 4.5°±0.2°, 9.0°±0.2° and 13.4°±0.2°.

More preferably, the main data of the X-ray powder diffraction pattern of fumarate salt crystalline form 2 is shown in Table 13.

**Table 13**

| Number | 2θ± 0.2 (°) | Interplanar Spacing [Å] | Relative Intensity(%) |
|---|---|---|---|
| 1 | 4.5 | 19.71 | 100 |
| 2 | 6.2 | 14.29 | 3.7 |
| 3 | 9.0 | 9.86 | 13.3 |
| 4 | 10.0 | 8.82 | 3.0 |
| 5 | 11.0 | 8.06 | 3.5 |
| 6 | 12.6 | 7.01 | 5.6 |
| 7 | 13.4 | 6.61 | 16.5 |
| 8 | 15.0 | 5.89 | 2.7 |
| 9 | 16.8 | 5.27 | 2.8 |
| 10 | 18.9 | 4.70 | 3.9 |
| 11 | 21.6 | 4.10 | 5.0 |
| 12 | 29.8 | 3.00 | 2.7 |

Preferably, the X-ray powder diffraction pattern of fumarate salt crystalline form 2 is approximately as shown in Figure 38.

Futher, the solubility of fumarate salt crystalline form 2 in water at 25°C is less than 0.05mg/mL.

Futher, the differential scanning calorimetry analysis shows the onset temperature of endothermic peak of the fumarate salt crystalline form 2 is about 182°C, and the peak temperature is about 194°C.

Futher, the thermal gravimetric analysis shows that fumarate salt crystalline form 2 has a weight loss of about 3.2% before 120°C.

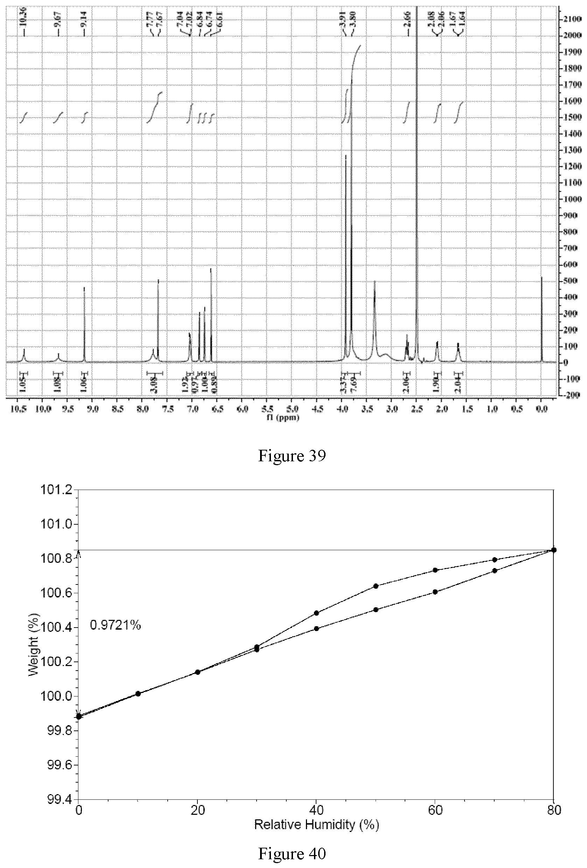

Further, fumarate salt crystalline form 2 has a ¹H-NMR spectrum substantially as shown in Figure 39.

According to the present invention, the purity of the maleate salt crystalline form 1, maleate salt crystalline form 2, methanesulfonate salt crystalline form 1, methanesulfonate salt crystalline form 2, besylate salt crystalline form 1, besylate salt crystalline form 2, hydrochloride salt crystalline form 1, phosphate salt crystalline form 1, L-tartrate salt crystalline form 1, L-malate salt crystalline form 1, citrate salt crystalline form 1, fumarate salt crystalline form 1 and fumarate salt crystalline form 2 are preferably greater than 50%, for example, greater than 85%, greater than 99%, or greater than 99.5%.

The present invention further provides methods of preparing the compound of Formula I and the salt forms thereof, which include maleate salt crystalline form 1, maleate salt crystalline form 2, methanesulfonate salt crystalline form 1, methanesulfonate salt crystalline form 2, besylate salt crystalline form 1, besylate salt crystalline form 2, hydrochloride salt crystalline form 1, phosphate salt crystalline form 1, L-tartrate salt crystalline form 1, L-malate salt crystalline form 1, citrate salt crystalline form 1, fumarate salt crystalline form 1 and fumarate salt crystalline form 2.

Wherein, the compound of Formula I can be prepared by the following route:

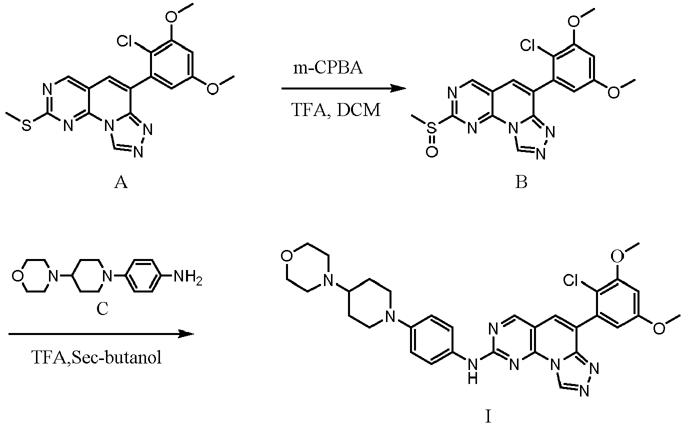

Wherein, the preparation methods of maleate salt crystalline form 1 are as follows:
The compound of Formula I was added into acetone/water, stirred at 60°C, the system was not clear, maleic acid was dissolved in water and added dropwise to the above suspension system, stirred to obtain a clear solution, and continued stirring for about 2 minutes until a solid was precipitated, stirring was continued at 60°C for 30 minutes, naturally cooled to room temperature and stirred for 4 days, filtered under reduced pressure, the solid was dried in vacuo for 24 hours to obtaine maleate salt crystalline form 1; or,
After the amorphous of maleate crystalline form 1 was added into dimethyl sulfoxide/ethanol, dimethyl sulfoxide/acetone, dimethyl sulfoxide/butanone, dimethyl sulfoxide/ethyl acetate, dimethyl sulfoxide/acetonitrile, trifluoroethanol /butanone, trifluoroethanol/methyl tert-butyl ether, trifluoroethanol/isopropyl acetate, trifluoroethanol/acetonitrile, water/methanol, water/ethanol, water/isopropanol, water/acetone or water/acetonitrile, the temperature was warmed up to 60°C to obtain a clear solution, filtered and stirred in a ice-salt bath. The solids were precipitated, and centrifuged immediately to obtain maleate crystalline Form 1. If there was no solid precipitated, stirred overnight at 4°C or the solution was evaporated out at room temperature to precipitated a solid, which was maleate crystalline form 1.

Wherein, the preparation methods of maleate salt crystalline form 2 are as follows:
Maleate salt crystalline form 1 was added into dimethyl sulfoxide and placed in a tetrahydrofuran system at room temperature, diffused and stand to crystallization, maleate salt crystalline form 2 was obtained; or,

Maleate salt crystalline form 1 was added into dimethyl sulfoxide/tetrahydrofuran, stirred at 60° C to clear, filtered, and the filtrate was stirred to precipitate a yellow solid, which was centrifuged at room temperature and dried under vacuum to obtain maleate salt crystalline form 2.

Wherein, the preparation methods of amorphous are as follows:
Maleate salt crystalline form 1 was added into trifluoroethanol/dichloromethane and stirred in a water bath at 60°C for 10 minutes. The system was not clear, the suspension was concentrated to dryness under reduced pressure at 40°C to obtain a dark yellow solid, which was amorphous of the maleate salt crystalline form 1.

Wherein, the preparation method of methanesulfonate salt crystalline form 1 is as follows:
Compound of Formula I was added acetone and water sequentially, stirred at 60°C in a water bath, the system was not clear, methanesulfonic acid was dissolved in water and added dropwise to the above suspension system, a clear solution was obtained. The clear solution was stirred at room temperature for 30 minutes, there was no precipitation, acetone was added dropwise and then a large amount of solids were precipitated, continued stirring at room temperature for 3 days, filtrated under reduced pressure, dried under vacuum at room temperature for 24 hours to obtain methanesulfonate salt crystalline form 1.

Wherein, the preparation method of methanesulfonate salt crystalline form 2 is as follows:
Compound of Formula I was added into acetone and water, stirred to clear at 60°C in a water bath, methanesulfonic acid was dissolved in acetone, and added dropwise to the above clear system, there was no precipitation, and stirring was continued at 60°C for 30 minutes, there was no precipitation. The mixture was naturally cooled to room temperature, stirred for 2 days, filtered under reduced pressure, and dried under vacuum at room temperature for 24 hours to obtain methanesulfonate salt crystalline form 2.

Wherein, the preparation method of besylate salt crystalline form 1 is as follows:
Compound of Formula I was added acetone, stirred at 60°C in a water bath, the system was not clear, benzenesulfonic acid was dissolved in water and added dropwise to the above suspension system at room temperature, the system was still not clear, continued stirring at room temperature for 5 days, filtrated under reduced pressure, dried under vacuum at room temperature for 24 hours, and benzenesulfonate salt crystalline form 1 was obtained.

Wherein, the preparation method of besylate salt crystalline form 2 is as follows:
Compound of Formula I was added acetone and water sequentially, stirred to clear at 60°C in a water bath, benzenesulfonic acid was dissolved in acetone, and added dropwise to the above clear system, there was no precipitation, stirring was continued at 60°C for 30 minutes, there was still no precipitation, naturally cooled to room temperature, stirred for 2 days to precipitate, filtered under reduced pressure, dried under vacuum at room temperature for 24 hours to obtain benzenesulfonate salt crystalline form 2.

Wherein, the preparation method of hydrochloride salt crystalline form 1 is as follows:
Compound of Formula I was added into acetone to disperse, stirred for 10 minutes at 50°C in a water bath, and then water was added, most of which was clear, concentrated hydrochloric acid was dissolved in water and added dropwise to the above suspension system, a large amount of white precipitate were precipitated, continued stirring for 30 minutes, naturally cooled to room temperature, stirred overnight, and continued stirring for 1 day, filtered under reduced pressure, dried under vacuum at 45°C for 3 hours to obtain hydrochloride salt crystalline form 1.

Wherein, the preparation method of phosphate salt crystalline form 1 is as follows:
Compound of Formula I was added acetone, stirred at 60°C in a water bath, the system was not clear, 85% phosphoric acid was dissolved in water and added dropwise to the suspension system at room temperature, the system was not clear, continued stirring at room temperature for 5 days, filtrated under reduced pressure, dried under vacuum at room temperature for 24 hours, to obtain phosphate salt crystalline form 1.

Wherein, the preparation method of L-tartrate salt crystalline form 1 is as follows:
Compound of Formula I was added isopropanol, stirred at 60°C for 10 minutes, the system was not clear, L-tartaric acid was dissolved in water, and added dropwise to the above suspension system, the system became clear after stirring, stirring was continued for 30 minutes, and then isopropanol was added dropwise, there was no precipitation, stirring was continued for 30 minutes, there was no precipitation, naturally cooled to room temperature, stirred overnight, precipitated solid, filtrated under reduced pressure, dried under vacuum at 45 °C for 3 hours to obtain L-tartrate salt crystalline form 1.

Wherein, the preparation method of L-malate salt crystalline form 1 is as follows:
Compound of Formula I was added isopropanol to disperse, stirred at 60°C for 10 minutes, the system was not clear, L-malic acid was dissolved in water, and added dropwise to the above suspension system, the system became clear after stirring, and stirring was continued for 10 minutes, and then isopropanol was added dropwise, there was no precipitation, stirring was continued for 30 minutes, there was still no precipitation, naturally cooled to room temperature, stirred overnight, precipitated solids, filtrated under reduced pressure, filter cake was rinsed with isopropanol and methyl tert-butyl ether 3 times, dried under vacuum at 45 °C for 3 hours to obtain L-malate salt crystalline form 1.

Wherein, the preparation method of citrate salt crystalline form 1 is as follows:
Compound of Formula I was added into isopropanol to disperse, stirred at 60°C for 10 minutes, the system was not clear, citric acid was dissolved in water, and added dropwise to the above suspension system, the system became clear after stirring, stirring was continued for 30 minutes, and then isopropanol was added dropwise, there was no precipitation, stirring was continued for 30 minutes, there was still no precipitation, naturally cooled to room temperature, stirred overnight, precipitated solids, filtrated under reduced pressure, filter cake was rinsed with isopropanol and methyl tert-butyl ether 3 times, dried under vacuum at 45 °C for 3 hours to obtain citrate salt crystalline form 1.

Wherein, the preparation method of fumarate salt crystalline form 1 is as follows:
Compound of Formula I was added tetrahydrofuran, stirred to clear at 60°C, fumaric acid was dissolved in tetrahydrofuran, and added dropwise to the clear system at room temperature, there was no precipitation, continued stirring for 4 days, precipitated solids, centrifuged, dried under vacuum at room temperature for 4 hours to obtain fumarate salt crystalline form 1.

Wherein, the preparation method of fumarate salt crystalline form 2 is as follows:
Compound of Formula I was added into isopropanol to disperse, stirred at 60°C for 10 minutes, the system was not clear, fumaric acid was dissolved in water, and added dropwise to the above suspension system, the system became clear after stirring, stirring was continued for 30 minutes, there was no precipitation, naturally cooled to room temperature and stirred overnight to precipitate a solid, continued stirring at room temperature for 1 day, filtrated under reduced pressure, dried under vacuum at 45 °C for 3 hours to obtain L-tartrate fumarate salt crystalline form 2.

The present invention further provides a pharmaceutical composition comprising a therapeutically effective amount of the salt form or the crystalline form of the present invention, and a pharmaceutically acceptable excipient, adjuvant or carrier. In the above mentioned composition, the weight ratio of the salt form or the crystalline form to the excipient, adjuvant or carrier can be within the range of 0.0001 to 10.

Secondly, the present invention also provides preferable embodiments of the above mentioned pharmaceutical composition.

Preferably, the pharmaceutical composition comprises a therapeutically effective amount of the salt form or the crystalline form of the present invention, in combination with at least one other active ingredient.

Preferably, the pharmaceutical composition is used in an oral administration.

Preferably, the pharmaceutical composition is used in a tablet or a capsule.

Preferably, the pharmaceutical composition comprises 0.01wt%-99wt% of the crystalline form of the present invention.

Preferably, the pharmaceutical composition comprises 0.05wt%-50wt% of the crystalline form of the present invention.

Preferably, the pharmaceutical composition comprises 0.1wt%-30wt% of the crystalline form of the present invention

The present invention further provides the use of the crystalline form or the pharmaceutical composition in the preparation of a medicament.

The present invention further provides preferable embodiments of the use:
Preferably, the use is treating, preventing, delaying or arresting the occurrence or progression in cancer or cancer metastasis.

Preferably, the use is the preparation of the medication for treating diseases mediated by FGFR.

Preferably, the disease is cancer.

Preferably, the cancer is selected from breast cancer, multiple myeloma, bladder cancer, endometrial cancer, stomach cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, intracranial tumors, hepatobiliary duct cell carcinoma, myelodysplastic syndrome, malignant glioma, prostate cancer, thyroid cancer, Schwann cell tumor, lung squamous cell carcinoma, lichenoid Keratosis, synovial sarcoma, skin cancer, pancreatic cancer, testicular cancer and liposarcoma.

Preferably, the use is to be an FGFR inhibitor.

Preferably, in the above-mentioned use, the FGFR contains FGFR1, FGFR2, FGFR3 or FGFR4.

The present invention also provides a method for administering to treatment subject a therapeutically effective amount of at least any one crystalline form or pharmaceutical composition to treating and/or preventing deseases mediated by FGFR.

Preferably, in the above-mentioned method, the FGFR contains FGFR1, FGFR2, FGFR3 or FGFR4.

Preferably, in the above-mentioned method, the desease mediated by FGFR is cancer.

Preferably, in the above-mentioned method, the cancer is selected from breast cancer, multiple myeloma, bladder cancer, endometrial cancer, stomach cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, intracranial tumors, hepatobiliary duct cell carcinoma, myelodysplastic syndrome, malignant glioma, prostate cancer, thyroid cancer, Schwann cell tumor, lung squamous cell carcinoma, lichenoid Keratosis, synovial sarcoma, skin cancer, pancreatic cancer, testicular cancer and liposarcoma.

The present invention also provides a method for treating cancer, comprising administering a therapeutically effective amount of at least any one crystalline form or pharmaceutical composition to treatment subject, and the cancer is breast cancer multiple myeloma, bladder cancer, endometrial cancer, stomach cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, intracranial tumors, hepatobiliary duct cell carcinoma, myelodysplastic syndrome, malignant glioma, prostate cancer, thyroid cancer, Schwann cell tumor, lung squamous cell carcinoma, lichenoid Keratosis, synovial sarcoma, skin cancer, pancreatic cancer, testicular cancer or liposarcoma.

Preferably, in the above-mentioned method, the treatment subject is human.

All the crystalline forms of the invention are substantially pure.

As used herein, the term "substantially pure" means that the content of the crystalline form is not less than 85 %, preferably not less than 95%, more preferably not less than 99%.

In present invention, the term "substantially" and "approximately" used in "has a X-ray powder diffraction pattern substantially as shown in Figure 1" or "the X-ray powder diffraction pattern is approximately as shown in Figure 1" means that the precise positions of the peaks in the diffractogram should not be construed as being absolute values. Because it is known by those skilled in the art that the 2θ values of the X-ray powder diffraction pattern may have errors depending on different measurement conditions (such as equipment and machine used) and different samples, a measurement error of a diffraction angle in an X-ray powder diffractogram is 5% or less, generally, a difference of ±0.2° from the given value is to be considered appropriate. It's should be understood that the relative intensities might fluctuate depending upon experimental conditions andd sample preparation such as the preferred orientation of the particles in the sample. The use of automatic or fixed divergence slits will also influence the relative intensity calculations. The intensities shown in the XRD curves included here are only exemplary and cannot be used for absolute comparition.

Those skilled in the art will understand that due to the changes in sample purity, sample preparation and measurement conditions (such as heating rate), the DSC measurement data may be undergo small changes. It's should be understood that alternative melting point readings may be given by other types of instruments or by using other conditions which is different from those described below. Thus, the endothermic diagrams cited in the present invention are not regarded as absolute values, and such measurement errors will be considered when interpreting the DSC date.

The term "therapeutically effective amount" as herein used, refers to the amount of a compound that, when administered to a subject for treating a disease, or at least one of the clinical symptoms of a disease or disorder, is sufficient to affect such treatment for the disease, disorder, or symptom. The "therapeutically effective amount" can vary with the compound, the disease, disorder, and/or symptoms of the disease or disorder, severity of the disease, disorder, and/or symptoms of the disease or disorder, the age of the subject to be treated, and/or the weight of the subject to be treated. An appropriate amount in any given instance can be apparent to those skilled in the art or can be determined by routine experiments. In the case of combination therapy, the "therapeutically effective amount" refers to the total amount of the combination objects for the effective treatment of a disease, a disorder or a condition.

The salt form or crystalline form of this invention can be combined as the active ingredient, and mixed with the pharmaceutical carrier to form the pharmaceutical composition. The carrier may take a wide variety of forms depending on the form of preparation desired for administarion, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the salt form or crystalline form of this invention may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

"Pharmaceutically acceptable carrier" refers to a conventional pharmaceutical carrier suitable for the desired pharmaceutical preparation, for example: diluents and excipients such as water, various organic solvents, etc.; such as starch, pregelatinized starch, sucrose, Fillers such as dextrin, mannitol, lactose, spray-dried lactose, microcrystalline cellulose, silicified microcrystalline cellulose, inorganic salts, etc.; such as starch syrup, dextrin, powdered sugar, syrup, mucilage, polyethylene glycol , Cellulose derivatives, alginate, gelatin, hydroxypropyl cellulose, copovidone and polyvinylpyrrolidone (PVP); wetting agents such as distilled water, ethanol and glycerin; such as dry starch, low substitution Disintegrants of hydroxypropyl cellulose, hydroxypropyl starch, agar, calcium carbonate, sodium bicarbonate, crospovidone, croscarmellose sodium, sodium carboxymethyl starch, etc.; such as quaternary ammonium compounds, Amino acid ethylamine derivatives, acetoacetates, β-dicarboxylic acid esters, aromatic acidic compounds, aliphatic acidic compounds, etc. absorption enhancers; such as sodium cetyl sulfate, sodium stearyl sulfate, dioctyl sulfate Sodium sulfosuccinate, sodium lauryl sulfonate, benzalkonium bromide, benzalkonium chloride, domefen, lecithin, cetyl alcohol, sodium lauryl sulfate, tween and spaan, etc. Surfactants; such as polyethylene glycol, carbomer, cellulose derivatives, glycerin gelatin, polyvinyl alcohol, cocoa butter, synthetic or fully synthetic fatty acid glycerides, polyvinyl alcohol 40 stearate, petrolatum, solid Paraffin, liquid paraffin, simethicone, lanolin, beeswax, and dolphin and other drug-carrying bases; absorption carriers such as kaolin and bentonite; such as talc, micronized silica gel, silicon dioxide, hydrogenated vegetable oil, dodecyl Lubricants such as magnesium sulfate, sodium lauryl sulfate, stearic acid, calcium stearate, magnesium stearate, sodium stearyl fumarate and polyethylene glycol. In addition, other pharmaceutically acceptable auxiliary materials can be added to the pharmaceutical composition, such as antioxidants, coloring agents, preservatives, pH regulators, hardeners, emulsifiers, propellants, dispersants, stabilizers, thickeners, Complexing agents, buffers, penetration enhancers, polymers, fragrances, sweeteners and dyes. Preferably, excipients suitable for the desired dosage form and desired mode of administration are used.

The term "disease" or "disorder" or "condition" refers to any disease, discomfort, illness, symptoms or indications.

### BRIEF DESCRIPTION OF THE DRAWINGS

Figure 1: An X-ray powder diffraction pattern of maleate salt crystalline form 1 of the compound represented by Formula I (this maleate salt crystalline form 1 prepeared by the samples with batch number 20180227-2 as raw material).
Figure 2: An X-ray powder diffraction pattern of maleate salt crystalline form 1 of the compound represented by Formula I (this maleate salt crystalline form 1 prepeared by the samples with batch number 20180123 as raw material).
Figure 3: Comparison of Figure 1 and Figure 2.
Figure 4: A differential scanning calorimetry thermogram of maleate salt crystalline form 1 of the compound represented by Formula I.
Figure 5: A Thermal gravimetric analysis thermogram of maleate salt crystalline form 1 of the compound represented by Formula I.
Figure 6: A ¹H-NMR spectrum of maleate solid crystalline form 1 of the compound represented by Formula I.
Figure 7: A X-ray powder diffraction pattern of maleate salt crystalline form 2 of the compound represented by Formula I.
Figure 8: A differential scanning calorimetry thermogram of maleate salt crystalline form 2 of the compound represented by Formula I.
Figure 9: A thermal gravimetric analysis thermogram of maleate salt crystalline form 2 of the compound represented by Formula I.
Figure 10: A X-ray powder diffraction pattern of amorphous of the compound represented by Formula I.
Figure 11: A hot-stage X-ray powder diffraction pattern of amorphous of the compound represented by Formula I.
Figure 12: A X-ray powder diffraction pattern of mesylate salt crystalline form 1 of the compound represented by Formula I.
Figure 13: A hot-stage X-ray powder diffraction pattern of mesylate salt crystalline form 1 of the compound represented by Formula I.
Figure 14: A thermal gravimetric analysis thermogram of mesylate salt crystalline form 1 of the compound represented by Formula I.
Figure 15: A differential scanning calorimetry thermogram of mesylate salt crystalline form 1 of the compound represented by Formula I.
Figure 16: A ¹H-NMR spectrum of mesylate salt crystalline form 1 of the compound represented by Formula I.
Figure 17: A X-ray powder diffraction pattern of mesylate salt crystalline form 2 of the compound represented by Formula I.
Figure 18: A hot-stage X-ray powder diffraction pattern of mesylate salt crystalline form 2 of the compound represented by Formula I.
Figure 19 : A thermal gravimetric analysis thermogram of mesylate salt crystalline form 2 of the compound represented by Formula I.
Figure 20: A differential scanning calorimetry thermogram of mesylate salt crystalline form 2 of the compound represented by Formula I.
Figure 31: A ¹H-NMR spectrum of mesylate salt crystalline form 2 of the compound represented by Formula I.
Figure 22: A X-ray powder diffraction pattern of besylate salt crystalline form 1 of the compound represented by Formula I.
Figure 23: A 1H-NMR spectrum of besylate salt crystalline form 1 of the compound represented by Formula I.
Figure 24: A X-ray powder diffraction pattern of besylate salt crystalline form 2 of the compound represented by Formula I.
Figure 25: A differential scanning calorimetry thermogram of besylate salt crystalline form 2 of the compound represented by Formula I.
Figure 26: A 1H-NMR spectrum of besylate salt crystalline form 2 of the compound represented by Formula I.
Figure 27: A X-ray powder diffraction pattern of hydrochloride salt crystalline form 1 of the compound represented by Formula I.
Figure 28: A X-ray powder diffraction pattern of phosphate salt crystalline form 1 of the compound represented by Formula I.
Figure 29: A differential scanning calorimetry thermogram of phosphate salt crystalline form 1 of the compound represented by Formula I.
Figure 30: A X-ray powder diffraction pattern of L-tartrate salt crystalline form 1 of the compound represented by Formula I.
Figure 31: A 1H-NMR spectrum of L-tartrate salt crystalline form 1 of the compound represented by Formula I.
Figure 32: A X-ray powder diffraction pattern of L-malate salt crystalline form 1 of the compound represented by Formula I.
Figure 33: A differential scanning calorimetry thermogram of L-malate salt crystalline form 1 of the compound represented by Formula I.
Figure 34: A ¹H-NMR spectrum of L-malate salt crystalline form 1 of the compound represented by Formula I.
Figure 35: A X-ray powder diffraction pattern of citrate salt crystalline form 1 of the compound represented by Formula I.
Figure 36: A ¹H-NMR spectrum of citrate salt crystalline form 1 of the compound represented by Formula I.
Figure 37: A X-ray powder diffraction pattern of fumarate salt crystalline form 1 of the compound represented by Formula I.
Figure 38: A X-ray powder diffraction pattern of fumarate salt crystalline form 2 of the compound represented by Formula I.
Figure 39: A ¹H-NMR spectrum of fumarate salt crystalline form 2 of the compound represented by Formula I.
Figure 40: A isothermal adsorption curve of maleate salt crystalline form 1 of the compound represented by Formula I.

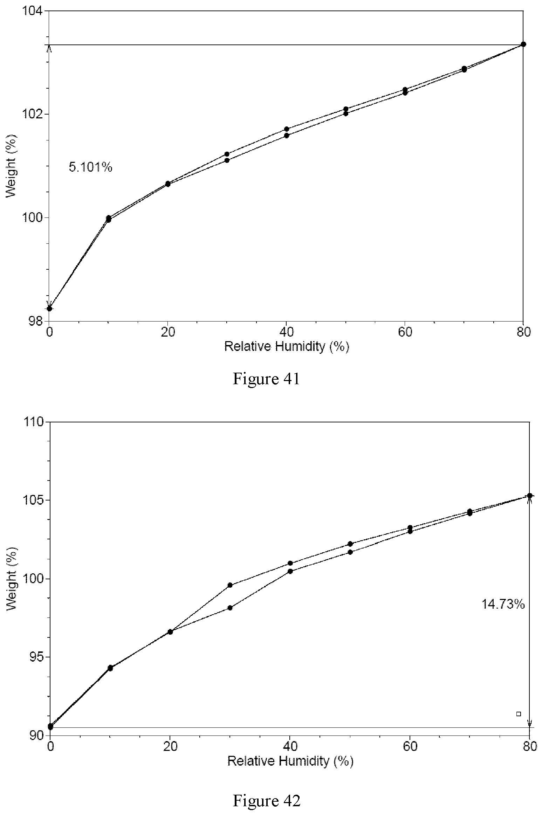

Figure 41: A isothermal adsorption curve of mesylate salt crystalline form 1 of the compound represented by Formula I.
Figure 42: A isothermal adsorption curve of mesylate salt crystalline form 2 of the compound represented by Formula I.

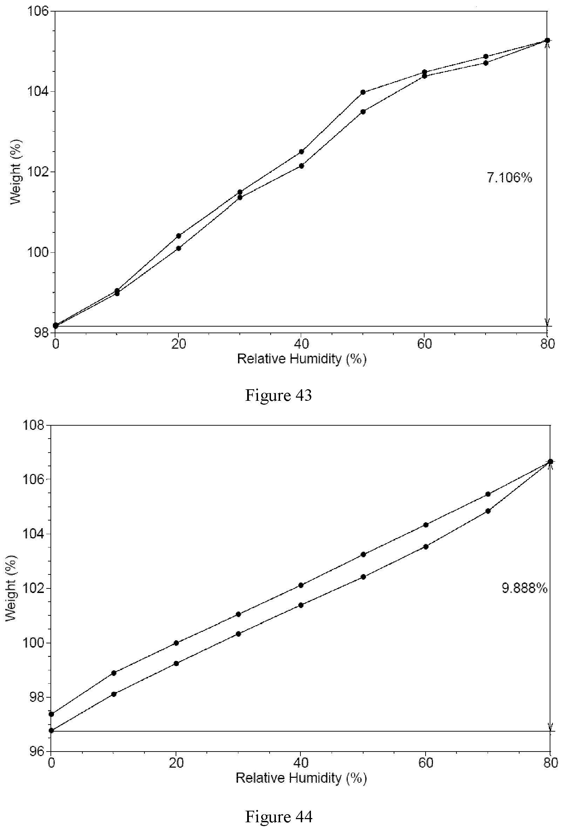

Figure 43: A isothermal adsorption curve of besylate salt crystalline form 1 of the compound represented by Formula I.
Figure 44: A isothermal adsorption curve of besylate salt crystalline form 2 of the compound represented by Formula I.

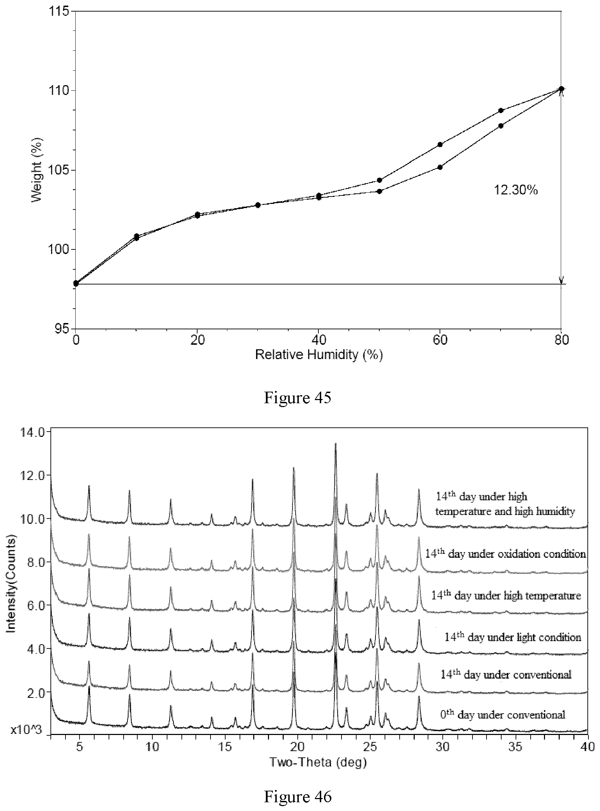

Figure 45: A isothermal adsorption curve of hydrochloride salt crystalline form 1 of the compound represented by Formula I.
Figure 46: A X-ray powder diffraction pattern of the influencing factors experiment of maleate salt crystalline form 1 of the compound represented by Formula I.

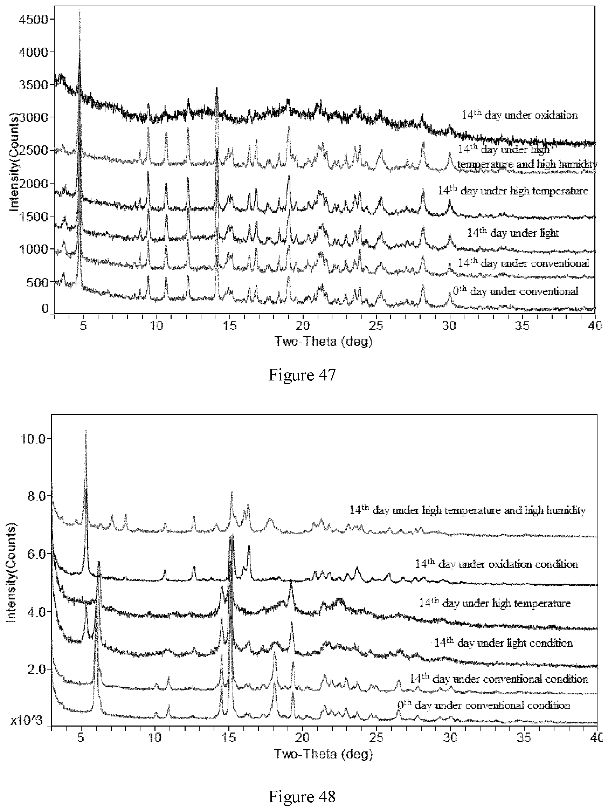

Figure 47: A X-ray powder diffraction pattern of the influencing factors experiment of mesylate salt crystalline form 1 of the compound represented by Formula I.
Figure 48: A X-ray powder diffraction pattern of the influencing factors experiment of the compound represented by Formula I.

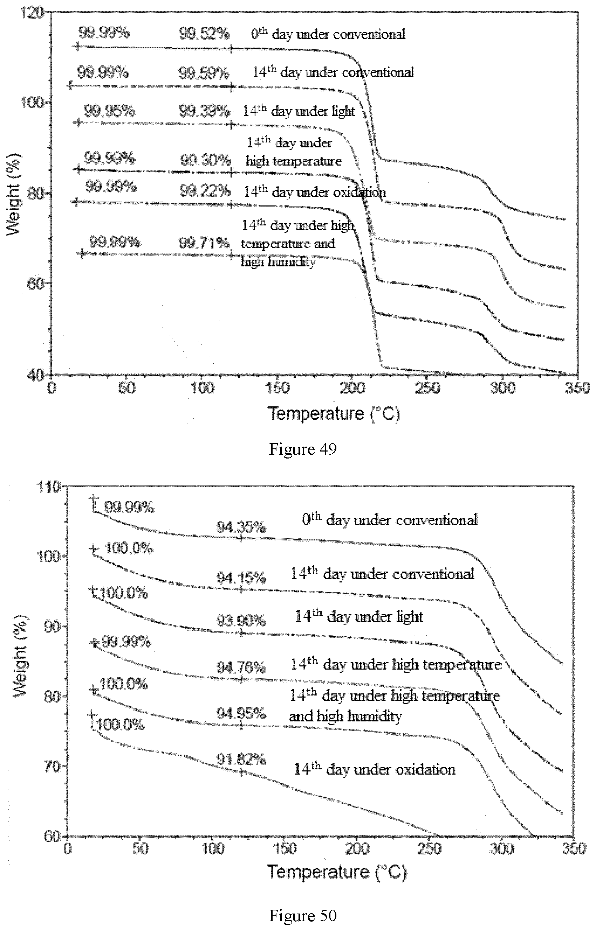

Figure 49: A thermal gravimetric analysis thermogram of the influencing factors experiment of maleate salt crystalline form 1 of the compound represented by Formula I.
Figure 50: A thermal gravimetric analysis thermogram of the influencing factors experiment of mesylate salt crystalline form 1 of the compound represented by Formula I.

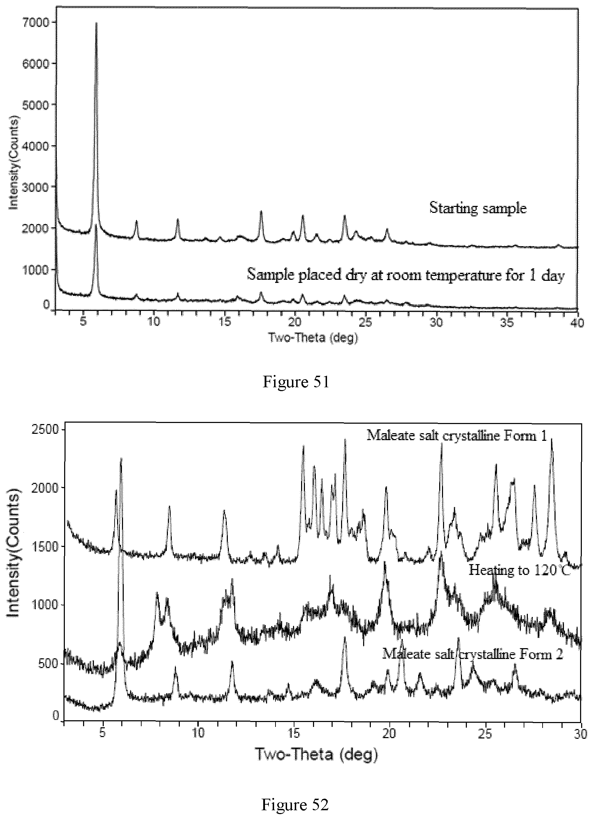

Figure 51: A X-ray powder diffraction pattern of the maleate solid crystalline form 2 of the compound represented by Formula I after dried for 1 day at room temperature.
Figure 52: A hot-stage X-ray powder diffraction pattern of maleate solid crystalline form 2 of the compound represented by Formula I.

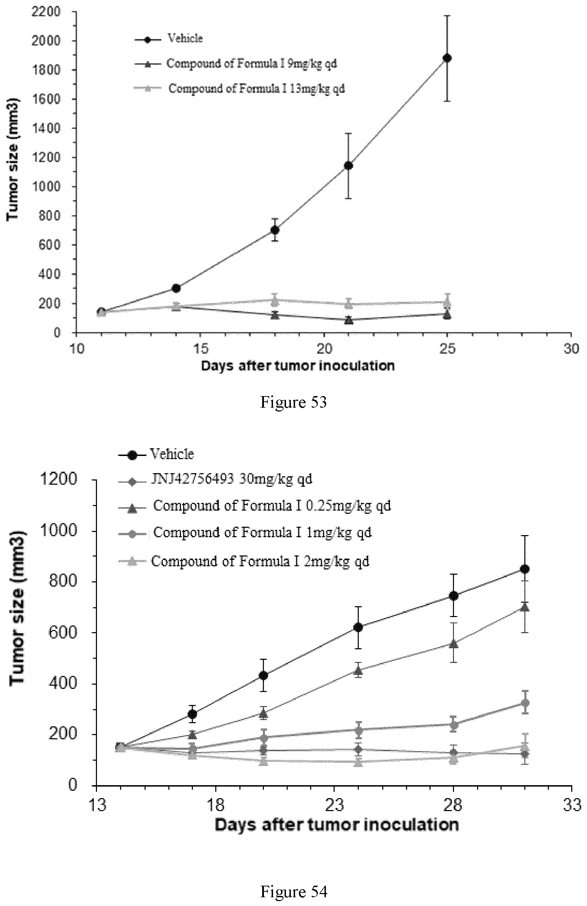

Figure 53: The anti-tumor activity of the compound represented by Formula I on the NCI-H1581 xenograft nude mouse model.
Figure 54: The anti-tumor activity of the compound represented by Formula I on the SNU-16 xenograft nude mouse model.

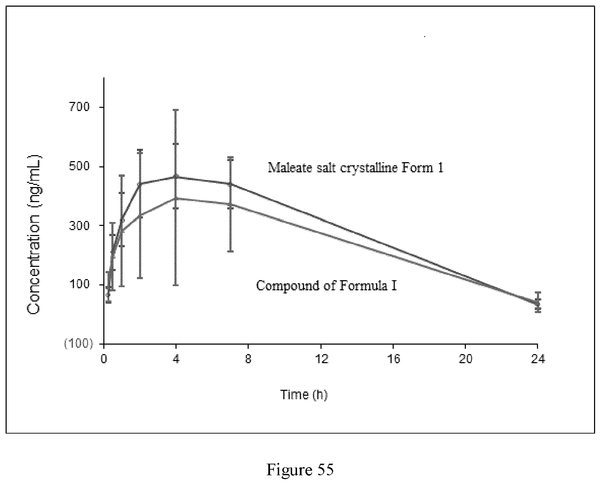

Figure 55: The plasma concentration-time curve of the compound represented by Formula I and its maleate solid crystalline form 1(administration dose 10mg/kg).

Unless otherwise specified, the detection instrument information and detection method parameters used in the present invention are as follows:

**Table 14**

| Device name | | X-ray powder diffractometer (XRD)& hot-stage XRD | |
|---|---|---|---|
| Equipment | | Bruker D8 Advance diffractometer | |
| Technical Specifications | | Kα radiation (40 kV, 40 mA) with a copper target wavelength of 1.54nm, θ-2θgoniometer, Mo monochromator,Lynxeye detector | |
| Calibrated substance | | Al₂O₃ | |
| Acquisition software | | Diffrac Plus XRD Commander | |
| Analysis software | | MDI Jade 6 | |
| Method parameters | Non reflective sample plate specification | | 24.6 mm diameter x1.0 mmthickness |
| | Variable temperature hot sample plate | | Copper plate |
| | Detection angle | | 3-40°2θ/3-30°2θ (hot-stage XRD) |
| | Step length | | 0.02°2θ |
| | speed | | 0.2s.step⁻¹ |
| | detection sample weight | | >2 mg |

**Table 15**

| Device name | Differential Thermal Analysis Scanner(DSC) |
|---|---|
| Instrument | TA Instruments Q200 DSC |
| Control software | Thermal Advantage |
| Analysis software | Universal Analysis |
| Sample plate | Aluminum crucible (with cover without perforation) |
| detection sample weight | 0.5-5 mg |
| Protective gas | Nitrogen |
| Gas flow rate | 4 0mL/m in |
| Common detection methods | Equilibrate at 25°C; Ramp 10°C/min to 300°C |

**Table 16**

| Device name | Thermogravimetric Analyzer(TGA) |
|---|---|
| Instrument | TA Instruments Q500TGA |
| Control software | Thermal Advantage |
| Analysis software | Universal Analysis |
| Sample plate | Platinum crucible |
| Sample detection amount | 1-10 mg |
| Protective gas | nitrogen |
| Gas flow rate | 4 0mL/m in |
| Detection method | Hi-Res sensitivity 3.0; |
| | Ramp 10.00°C/min res 5.0 to 120.00°C; |
| | Ramp 10.00°C/min to 350°C |

**Table 17**

| | | |
|---|---|---|
| Device name | Dynamic Vapor Sorption(DVS) | |
| Instrument | TA Instruments Q5000TGA | |
| Control software | Thermal Advantage | |
| Analysis software | Universal Analysis | |
| Sample plate | Platinum crucible | |
| detection sample weight | 1-10 mg | |
| Protective gas | nitrogen | |
| Gas flow rate | 10mL/min | |
| Detection method | Equilibrate at 25°C; Humidity 0%; Isothermal for 60 min; Abort next iso if weight (%) < 0.0100 for 15.00 min; step humidity 10% every 60 min to 80.00%; Abort next iso if weight(%) < 0.0100 for 15.00 min; step humidity 10% every 60 min to 0.00% | |
| Judgment standard | non-hygrosc opic | Not higher than 0.2% |
| | Slightly-hygroscopic | Higher than 0.2%,but not higher than 2.0% |
| | hygroscopic | Higher than 2%,but not higher than 15% |
| | Very hygroscopic | Higher than 15% |

**Table 18**

| | |
|---|---|
| Instrument | Nuclear Magnetic Resonance(NMR) |
| model | Bruker Ascend 500 |
| Detection type | NMR Hydrogen spectrum |
| parameter | Full-frequency excitation,single pulse with 30 ppm spectrum width, 16 scans with 30o angle excitation,digital orthogonal detection,temperature control 298K |

**Table 19**

| | | | |
|---|---|---|---|
| Instrument | High performance liquid chromatography(HPLC) | | |
| model | Ultimate 3000 | | |
| Column | Poroshell 120 EC-C18 (2.7 um 150×2.1 mm) | | |
| Flow rate | 0.4 ml/min | | |
| Detection wavelength | 231 nm | | |
| Injection volume | 2 uL | | |
| operation time | 45 min | | |
| Sample solvent | 0.1% Trifluoroacetic acid-50% acetonitrile aqueous solution | | |
| Injection concentration | 0.2 mg/ml | | |
| Mobile phase | Mobile phase A: 0.1% trifluoroacetic acid aqueous solution | | |
| | Mobile phase B: acetonitrile | | |
| Execute gradient | time(min) | A(%) | B(%) |
| | 0 | 90 | 10 |
| | 30 | 60 | 40 |
| | 40 | 10 | 90 |
| | 40.1 | 90 | 10 |
| | 45 | 90 | 10 |
| Remarks | 1) Purity calculation method: main peak area/ total peak area × 100%; where total peak area refer to the sum area | | |
| | of all peak which has an area greater than 0.02% of the main peak. | | |
| | 2) The single sample and single needle used in this HPLC related substance detection, the reported data value of the related substance is accurate to two decimal places. | | |

**Table 20. Solubility determination**

| method | Visual inspection |
|---|---|
| Specific operation method | Weigh a known amount of sample at 25°C, add solvent to the sample in batches, stir or ultrasonically aid dissolution until the sample is visually dissolved and record the amount of solvent consumed. If the sample is still not dissolved at a specific concentration, its solubility is expressed as "<" the specific concentration. |

**Table 21**

| | |
|---|---|
| Instrument | constant temperature and humidity chamber |
| model | SHH-250SD |
| Manufacturer | Chongqing Yong Sheng Laboratory Instrument Factory. |
| temperature | 40°C |
| Relative humidity | 75% |
| Specific operation method | The sample is placed in a watch glass and opened and placed in a light-tight condition |

**Table 22**

| | |
|---|---|
| Instrument | High Temperatureoven |
| model | DHG-9053A |
| Manufacturer | Shanghai Jing Hong Laboratory Instrument Co.,Ltd. |
| Temperature setting | 60 °C |
| Specific operation method | The sample is placed in a watch glass and opened and placed in a light-tight condition |

**Table 23**

| | |
|---|---|
| Instrument | irradiation box |
| model | SHH-100GD-2 |
| Manufacturer | Chongqing Yong Sheng Laboratory Instrument Factory |
| temperature | 25 °C |
| Illuminance | 4500 lux±500 lux |
| Specific operation method | The sample is placed in a watch glass and opened and placed in a light-tight condition |

### EXAMPLES

The present invention is further exemplified, but not limited, by the following examples that illustrate the invention. In the examples of the present invention, unless expressly stated, the technique or method is a conventional technique or method in the art.

### Abbreviation:

API: Active Pharmaceutical Ingredients;
ATCC: American Type Culture Collection;
DCM: dichloromethane;
¹³C-NMR: 13C Nuclear Magnetic Resonance;
DMSO: dimethyl sulfoxide;
h: hour;
¹H-NMR: 1H Nuclear Magnetic Resonance;
HPLC: High Performance Liquid Chromatography;
LC/MS: Liquid Chromatography-Mass Spectrometry;
m-CPBA: meta-chloroperoxybenzoic acid;
min: minute;
PO: oral administration;
RH: Relative Humidity;
RRT: Relative Retention Time;
TFA: trifluoroacetic acid;
TGA: thermogravimetric analysis;
UPLC: Ultra Performance Liquid Chromatography;
XRD: X-ray powder diffraction.

### Preparation of intermediate M1

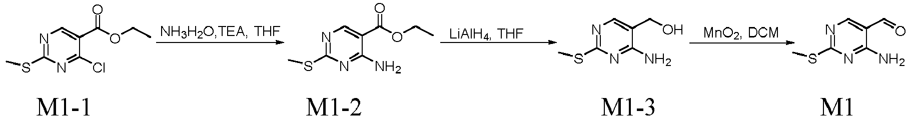

### Step1: Preparation of compound M1-2

500g of compound M1-1, 651g of TEA and 782ml of ammonia (25%) were dissolved in 2.4L of THF, stirred at RT for 12hrs. The reaction mixture was diluted with water, extracted with EA, washed with water, dried over anhydrous Na₂SO₄, and concentrated under reduced pressure to obtain 450g of compound M1-2 with a yield of 98.3%. It was used directly in the next step without purification.
LC-MS[M+H⁺]214.

### Step 2: Preparation of compound M1-3

Under nitrogen protection at -20°C, 570ml of LiAlH₄ (2.5N, suspended in THF) was added dropwise with stirring to a mixture of 250g of compound M1-2 dissolved in 2L THF, the reaction mixture was stirred at -10°C for 3hrs, 50ml of water was added to terminate the reaction, below 15°C, 50ml of 15% NaOH solution was added under stirring, and then 150ml of water was added. The mixture was filtered, the filter cake was washed with EA, the filtrate was collected and concentrated under reduced pressure to obtain 180 g of compound M1-3 with a yield of 89.7%, it was used directly in the next step without purification.
LC-MS [M+H⁺] 172.1.

### Step 3: Preparation of compound M1

A mixture of 320g M1-3, 1465g MnO₂ and 3L DCM was stirred at RT for 12hrs,filtered, the filter cake was washed with DCM, the filtrate was collected and concentrated under reduced pressure to obtain 261g of M1 with a yield of 82.5%, ,it was used directly in the next reaction without purification.

### Synthesis of compound A

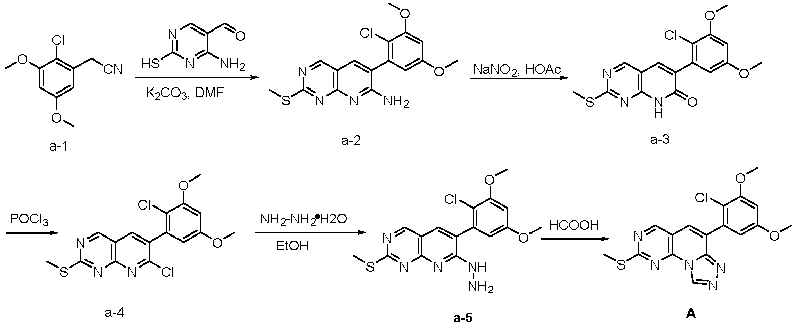

### Step 1: Preparation of compound a-2

A mixture of 16.77g a-1, 11.17g M1, 27.33g K₂CO₃ and 150ml DMF was stirred at 110°C for 12hrs. The reaction mixture was cooled to RT, poured into ice water, filtered, the filter cake was washed with water and dried under reduced pressure to obtain 17.21g of compound a-2 as a yellow solid, it was used directly in the next step without purification.
LC-MS [M+H⁺] 363.1.

### Step 2: Preparation of compound a-3

16.75g of compound a-1 was dissolved in 50ml of acetic acid, stirred at RT, 15.97g of NaNO₂ was added in portions, the reaction mixture was stirred at 70°C for 3hrs, cooled to RT, poured into ice water, filtered, the filter cake was washed with water, and dried under reduced pressure to obtain 15.21g of compound a-3 as light yellow solid.
LC-MS [M+H⁺] 364.0.

### Step 3: Preparation of compound a-4

A mixture of 2.71g of compound a-3 and 50ml of phosphorus oxychloride was stirred at 100°C for 3hrs, the reaction solution was concentrated to remove most of the phosphorus oxychloride, and the residue was quenched with ice water. The pH was adjusted to 8 with saturated NaHCO₃, the mixture was extracted with EA, washed with saturated sodium chloride, dried over anhydrous Na₂SO₄, and concentrated under reduced pressure to obtain 3.01 g of compound a-4 as a white solid, which was used directly in the next step without purification.

### Step 4: Preparation of compound a-5

A mixture of 500 mg of compound a-4, 818 mg of hydrazine hydrate (80%) and 25 ml of ethanol was refluxed for 2 hrs, concentrated under reduced pressure to obtain 520 mg of crude compound a-5 as a yellow solid, which was used directly in the next step without purification.
LC-MS [M+H⁺] 378.1.

### Step 5: Preparation of compound A

A mixture of 520 mg of compound a-5 and 25 ml of formic acid was refluxed for 1 h, the reaction mixture was diluted with water, filtered, and the filter cake was washed with water to obtain 310 mg of compound A as a pale yellow solid with a yield of 58%.
LC-MS [M+H⁺] 388.1.

### Example 1: Synthesis of the compound of Formula I:

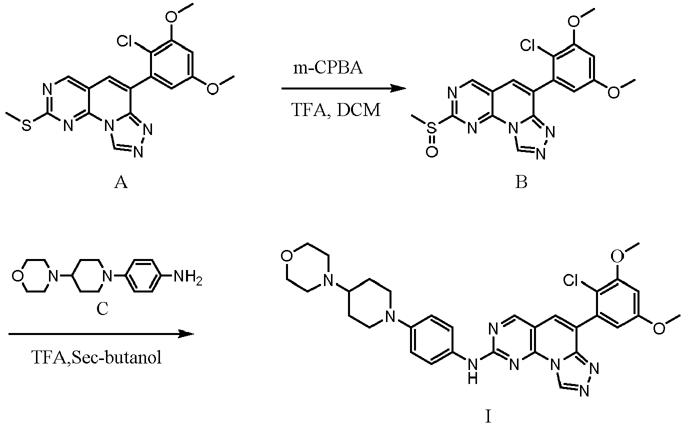

### Step 1: Synthesis of compound B

Compound A (100.01 g), DCM (2000 mL) and TFA (130 mL) was sequentially added into a 10L three-neck flask with stirring, and stirring was continued until the system was clear. The reaction system was cooled to 0°C to -5°C, m-CPBA was dissolved in ethyl acetate and then added dropwise to the above reaction system, the addition was completed after 30 minutes, and the temperature was maintained at 0°C to 5°C for 1.5 hours. The reaction was monitored by thin layer chromatography (DCM: methanol=15:1), and sampled for LC-MS to detected the completion. 103.98 g of anhydrous sodium sulfite was dissolved in 2600 mL of water and added dropwise to the reaction system to quench the reaction. After 10 minutes, the dripping is completed, stand still and separate. 1000 mL of water was added to the organic phase, stirred for 5 min, stand still and separate. The organic phase was concentrated under reduced pressure. The obtained residue was added 1000 mL of ethyl acetate and slurried for 1 h, and filtered. The filter cake was added 1000 mL of anhydrous methanol and slurried for 1 hour and filtered. The filter cake was dried under vacuum at 45° C for 14 hours to obtain 78.57 g of light yellow solid B with a yield of 75.4%.

### Step 2: Synthesis of compound of Formula I

Compound B (77.52g), sec-butanol (1550mL), compound C (60.21g) and TFA (39mL) were sequentially added into a 10L three-necked flask with stirring, and start heating to warm to reflux for 6 hours, the reaction was monitored by thin layer chromatography (DCM: methanol=15:1), and sampled for LC-MS to detected the completion. After cooling, the reaction system was concentrated under reduced pressure, and the concentrated residue was added to 1162 mL of anhydrous methanol and slurried for 30 min to 1 h, and filtered. The filter cake was added 775 mL of anhydrous methanol and slurried for 10 to 30 minutes, and filtered. The filter cake was sampled for UPLC to test purity. The filter cake was added anhydrous methanol (1077mL), DCM (5385mL) and TFA(179mL), stirried to dissolve and filter. 428.10 g of anhydrous potassium carbonate was dissolved in 4200 mL of water, added dropwise to the filtrate, stirred, and the pH was adjusted to alkaline, stand still and separated. The organic phase was concentrated under reduced pressure, and the obtained solid was added 898 mL of anhydrous methanol and slurried for 1 h, and filtered. The filter cake was dried under vacuum at 45° C for 12 hours to obtain 105.36 g of the compound of Formula I with a yield of 91.3%.

### Example 2: The first preparation method of maleate salt crystalline form 1 of the compound of Formula I

4.0g of the compound of Formula I was added 200mL of acetone and 36mL of water, stirred at 60°C, the system was not clear, 2.318g of maleic acid was dissolved in 4.0mL of water, and added dropwise to the above suspension system, the system was stirred to dissolve and stirring was continued for about 2 minutes, the solid was precipitated, continued stirring at 60°C for 30 minutes, naturally cooled to RT, stirred for 4 days, filtered under reduced pressure, and dried in vacuo at room temperature for 24 hours to obtain 5.336 g of maleate salt crystalline form 1 of the compound of Formula I.

### Example 3: The second preparation method of maleate salt crystalline form 1 of the compound of Formula I

40.03 g of the compound of Formula I was added into 800 mL of anhydrous methanol, 400 mL of water was added, and the temperature was raised to 50°C. 8.50 g of maleic acid was dissolved in 40 mL of water and added dropwise to the above reaction system. The system was gradually dissolved and filtered while hot. The filtrate was transferred to a 5L three-necked flask, 1200 mL of anhydrous methanol was added with stirring, and the temperature was rasied to 50°C. 14.69 g of maleic acid was dissolved in 60 mL of water and added dropwise to the reaction system. After the dropwise addition, the reaction was naturally cooled and stirred overnight. Filtered and the filter cake was rinsed with anhydrous methanol. The filter cake was dried under vacuum at 60° C for 20 hours to obtain 53.27 g of light yellow solid, which is maleate salt crystalline form 1 of the compound of Formula I, and the yield was 96.0%.

### Example 4: The first preparation method of maleate salt crystalline form 2 of the compound of Formula I

About 10 mg of maleate saltmaleate saltmaleate salt crystalline form 1 was added into 0.1 mL of dimethyl sulfoxide, and placed in a tetrahydrofuran system at room temperature for diffusion and standing for crystallization, and the maleate salt crystalline form 2 was obtained.

### Example 5: The second preparation method of maleate salt crystalline form 2 of the compound of Formula I

About 10 mg of maleate salt crystalline form 1 was sequentially added 0.1 mL of dimethyl sulfoxide and 5.0 mL of tetrahydrofuran, stirred to clear at 60°C, filtered, and the filtrate was placed in an ice-salt bath and stirred to precipitate a yellow solid, centrifuged at room temperature and dried under vacuum, to obtain the maleate salt crystalline form 2 was obtained.

### Example 6: Preparation of methanesulfonate salt crystalline form 1 of the compound of Formula I

400 mg of the compound of Formula I was sequentially added 20 mL of acetone and 2 mL of water, stirred at 60°C in a water bath, the system was not clear, 70.35 mg of methanesulfonic acid was dissolved in 2.0 mL of water, and added dropwise to the above suspension system, and then the system was clear. The system was removed to room temperature and stirred for 30 minutes, there was no precipitation, 20 mL of acetone was added dropwise to the system, and then a large amount of solid was precipitated, continued stirring at room temperature for 3 days, filtered under reduced pressure, and dried under vacuum at room temperature for 24 hours to obtain 342 mg of methanesulfonate salt crystalline form 1.

### Example 7: Preparation of methanesulfonate salt crystalline form 2 of the compound of Formula I

200 mg of the compound of Formula I was added 10 mL of acetone and 2 mL of water, stirred to clear at 60°C in a water bath, 96 mg of methanesulfonic acid was dissolved in 0.2 mL of acetone, and added dropwise to the above clear system, there was no precipitation, stirring was continued at 60°C for 30 minutes, there was no precipitation, naturally cooled to room temperature, stirred for 2 days, filtered under reduced pressure, and dried under vacuum at room temperature for 24 hours to obtain 160 mg of methanesulfonate salt crystalline form 2.

### Example 8: Preparation of besylate salt crystalline form 1 of the compound of Formula I

100 mg of the compound of Formula I was added 20 mL of acetone, stirred at 60°C in a water bath, the system was not clear, 28.94 mg of benzenesulfonic acid was dissolved in 1.0 mL of water and added dropwise to the above suspension system at room temperature, the system was still not clear, continued stirring at room temperature for 5 days, filtered under reduced pressure, and dried under vacuum at room temperature for 24 hours to obtain 54mg of besylate salt crystalline form 1.

### Example 9: Preparation of besylate salt crystalline form 2 of the compound of Formula I

200 mg of the compound of Formula I was added 10 mL of acetone and 2.0 mL of water sequentially, stirred to clear at 60 °C in a water bath, 158 mg of benzenesulfonic acid was dissolved in 0.2 mL of acetone, and added dropwise to the above clear system, there was still precipitation, the stirring was continued at 60°C for 30 minutes, there was still no precipitation, naturally cooled to room temperature, stirried for 2 days, solid was precipitated, filtered under reduced pressure and dried under vacuum at room temperature for 24 hours to obtain 141 mg of besylate salt crystalline form 2.

### Example 10: Preparation of hydrochloride salt crystalline form 1 of the compound of Formula I

200 mg of the compound of Formula I was added 10 mL of acetone to disperse, stirred at 50°C in a water bath for 10 minutes, then 1.8 mL of water was added, most of it was dissolved, 101.09 mg of concentrated hydrochloric acid was dissolved in 0.2 mL of water and added dropwise to the above suspension system, a large amount of white solids were precipitated, stirring was continued for 30 minutes, and then naturally cooled to room temperature, stirred overnight, and continued stirring for 1 day, filtered under reduced pressure, dried under vacuum at 45°C for 3 hours to obtain 158 mg of hydrochloride crystalline form 1.

### Example 11: Preparation of phosphate salt crystalline form 1 of the compound of Formula I

100mg of the compound of Formula I was added 20mL of acetone, stirred at 60° C in a water bath, the system was not clear, 21.10mg of 85% phosphoric acid was dissolved in 1.0mL of water and added dropwise to the above suspension system at room temperature, the system was still not clear, continued stirring for 5 days, filtered under reduced pressure, and dried under vacuum at room temperature for 24 hours to obtain 51 mg of phosphate salt crystalline form 1.

### Example 12: Preparation of L-tartrate salt crystalline form 1 of the compound of Formula I

150 mg of the compound of Formula I was added 2.5 mL of isopropanol, stirred at 60°C for 10 minutes, the system was not clear, 112 mg of L-tartaric acid was dissolved in 2.5 mL of water and added dropwise to the above suspension system, the system was stirred to clear, and continued stirring for 10 minutes, 7.5mL isopropanol was added dropwise, there was no precipitation, stirring was continued for 30 minutes, there was still no precipitation, naturally cooled to room temperature, stirred overnight, a solid was precipitated, filtered under reduced pressure, and dried under vacuum at 45°C for 3 hours to obtain 120mg of L-tartrate salt crystalline form 1.

### Example 13: Preparation of L-malate salt crystalline form 1 of the compound of Formula I

150 mg of the compound of Formula I was added 2.5 mL of isopropanol to disperse and stirred at 60°C for 10 minutes, the system was not clear, 100 mg of L-malic acid was dissolved in 2.5 mL of water and added drowpwise to the above suspension system, the system was stirred to clear, continued stirring for 10 minutes, 7.5mL of isopropanol was added dropwise, there was no precipitation, continued stirring for 30 minutes, there was still no precipitation, naturally cooled to room temperature, stirred overnight, a solid was precipitated, filtrated under vacuum, filter cake was rinsed three times with isopropanol and methyl tert-butyl ether, dried under vacuum at 45°C for 3 hours to obtain 120 mg of L-malate salt crystalline form 1.

### Example 14: Preparation of citrate salt crystalline form 1 of the compound of Formula I

150 mg of the compound of Formula I was added 2.5 mL of isopropanol to disperse and stirred at 60°C for 10 minutes, the system was not clear, 144 mg of citric acid was dissolved in 2.5 mL of water and added drowpwise to the above suspension system, the system was stirred to clear, continued stirring for 10 minutes, 7.5mL isopropanol was added dropwise, there was no precipitation, continued stirring for 30 minutes, there was still no precipitation, naturally cooled to room temperature, stirred overnight, a solid was precipitated, filtrated under vacuum, filter cake was rinsed three times with isopropanol and methyl tert-butyl ether, and dried under vacuum at 45°C for 3 hours to obtain 124 mg of L-malate salt crystalline form 1.

### Example 15: Preparation of fumarate salt crystalline form 1 of the compound of Formula I

20 mg of the compound of Formula I was added 5.0 mL of tetrahydrofuran, stirred to clear at 60°C, 4.25 mg of fumaric acid was dissolved in 0.2 mL of tetrahydrofuran and added dropwise to the above solution system at room temperature, there was no precipitation, continued stirring at room temperature for 4 days, a solid was precipitated, centrifuged, and dried under vacuum at room temperature for 4 hours to obtain fumarate salt crystalline form 1.

### Example 16: Preparation of fumarate salt crystalline form 2 of the compound of Formula I

150 mg of the compound of Formula I was added 2.5 mL of isopropanol to disperse and stirred at 60°C for 10 minutes, the system was undissolved, 87 mg of fumaric acid was dissolved in 5 mL of water and added drowpwise to the above suspension system, the system was stirred to clear, continued stirring for 30 minutes, there was no precipitation, naturally cooled to room temperature, stirred overnight, a solid was precipitated, stirring was continued for 1 day at RT, filtrated under vacuum, filter cake was dried under vacuum at 45°C for 3 hours to obtain 124 mg of fumarate salt crystalline form 2.

### Example 17 XRD analysis result of maleate salt crystalline form 1 of the compound of Formula I

Two batches of samples of the compound represented by Formula I in different batch lots (batch numbers: 20180227-2 and 20180123) were obtained according to the preparation method provided in Example 1 of the present invention, and then obtained two batches of maleate salt crystalline form 1 in different batch lots according to the preparation method of Example 2, and characterized them separately, the XRD spectra are shown in Figure 1 to Figure 3, the spectral analysis data are shown in Table 24 and Table 25 respectively.

**Table 24 The XRD spectral analysis data of maleate salt crystalline form 1 prepared from sample with batch number 20180227-2 as raw material**

| Number | 2θ± 0.2 (°) | Relative Intensity(%) |
|---|---|---|
| 1 | 5.6 | 50.9 |
| 2 | 8.4 | 46.9 |
| 3 | 10.7 | 3.5 |
| 4 | 11.2 | 47.5 |
| 5 | 12.6 | 6.5 |
| 6 | 13.3 | 9.4 |
| 7 | 14.0 | 14.9 |
| 8 | 15.3 | 93.1 |
| 9 | 15.6 | 36.3 |
| 10 | 15.9 | 75.9 |
| 11 | 16.3 | 39.1 |
| 12 | 17.0 | 48.2 |
| 13 | 17.5 | 83.4 |
| 14 | 17.9 | 17.9 |
| 15 | 18.3 | 22.9 |
| 16 | 18.5 | 43.0 |
| 17 | 19.7 | 80.5 |
| 18 | 20.1 | 25.2 |
| 19 | 20.7 | 7.2 |
| 20 | 21.0 | 3.8 |
| 21 | 21.9 | 11.3 |
| 22 | 22.6 | 93.2 |
| 23 | 23.3 | 40.9 |
| 24 | 24.6 | 7.7 |
| 25 | 25.0 | 10.1 |
| 26 | 25.4 | 63.1 |
| 27 | 26.3 | 48.2 |
| 28 | 26.9 | 6.7 |
| 29 | 27.5 | 53.4 |
| 30 | 28.3 | 100 |
| 31 | 29.1 | 5.5 |
| 32 | 30.4 | 11.9 |
| 33 | 31.1 | 4.6 |
| 34 | 31.7 | 5.2 |
| 35 | 32.5 | 6.3 |
| 36 | 33.6 | 7.8 |
| 37 | 34.3 | 5.8 |
| 38 | 35.5 | 4.2 |
| 39 | 36.2 | 2.7 |
| 40 | 37.1 | 3.9 |
| 41 | 37.7 | 2.7 |

**Table 25 The XRD spectral analysis data of maleate salt crystalline form 1 prepared from sample with batch number 20180123 as raw material**

| Number | 2θ± 0.2 (°) | Relative Intensity(%) |
|---|---|---|
| 1 | 5.7 | 42.6 |
| 2 | 8.5 | 39.2 |
| 3 | 11.3 | 29.6 |
| 4 | 12.6 | 2.2 |
| 5 | 13.5 | 3.1 |
| 6 | 14.1 | 13.9 |
| 7 | 15.5 | 3.3 |
| 8 | 15.7 | 11.4 |
| 9 | 16.1 | 2.3 |
| 10 | 16.4 | 2.5 |
| 11 | 16.9 | 54.6 |
| 12 | 17.6 | 3.0 |
| 13 | 18.3 | 1.4 |
| 14 | 18.6 | 3.2 |
| 15 | 19.8 | 73.1 |
| 16 | 20.1 | 2.5 |
| 17 | 20.7 | 2.2 |
| 18 | 22.0 | 2.2 |
| 19 | 22.6 | 100.0 |
| 20 | 23.4 | 25.3 |
| 21 | 24.7 | 4.7 |
| 22 | 25.0 | 15.4 |
| 23 | 25.5 | 59.5 |
| 24 | 26.1 | 18.1 |
| 25 | 27.0 | 2.3 |
| 26 | 27.5 | 3.1 |
| 27 | 28.4 | 44.5 |
| 28 | 30.3 | 2.4 |
| 29 | 30.6 | 1.8 |
| 30 | 31.3 | 2.4 |
| 31 | 31.8 | 2.7 |
| 32 | 33.6 | 1.4 |
| 33 | 34.4 | 3.4 |
| 34 | 36.3 | 1.7 |
| 35 | 36.8 | 1.1 |
| 36 | 37.1 | 1.7 |

According to the XRD spectrum analysis data and the comparison chart in Figure 3, those skilled in the art should understand that the relative intensity of each peak of the two batches of maleate salt crystalline form 1 which recorded in the analysis data table and in the XRD spectrum can be changed due to many factors (such as the crystal orientation in the X-ray beam and the purity of the analyte, etc.). The peak position can also be slightly shifted due to the change in sample weight; however, the crystalline form of the two batches is essentially the same. It should be understood that the XRD pattern of the maleate salt crystalline form 1 of the compound of Formula I provided by the present invention is not limited to the X-ray powder diffraction pattern shown in FIG. 1 or FIG. 2, and the crystalline forms which have X-ray powder diffraction pattern that are basically the same as those shown in Figure 1 or Figure 2 fall within the scope of the present invention.

### Example 18 Hygroscopicity Assessment of crystalline form of the comound of Formula I

The hygroscopicity of the sample (the sample is a variety of crystal forms shown in Table 26) was tested using dynamic vapor sorption (DVS) instrument, and the water uptake of the sample at 0%RH (relative humidity) to 80%RH was tested. The test results were shown in Figure 40-45 and Table 26.

**Table 26 weight change of the samples between 0%RH to 80%RH**

| Crystalline form | water uptake |
|---|---|
| compound of Formula I | 15.3% |
| maleate salt crystalline Form 1 | 0.97% |
| methanesulfonate salt crystalline Form 1 | 5.1% |
| methanesulfonate salt crystalline Form 2 | 14.7% |
| besylate salt crystalline Form 1 | 7.1% |
| besylate salt crystalline Form 2 | 9.9% |
| hydrochloride salt crystalline Form 2 | 12.3% |

It can be seen from the test results that the maleate crystalline form 1 has outstanding advantages in terms of hygroscopicity. Compared with the compound of structural formula I, the hygroscopicity of maleate crystalline form 1 is greatly reduced. Methanesulfonate crystalline form 1 also has obvious advantages, and other crystalline forms have relatively high hygroscopicity.

### Example 19: Stability assessment of the compound of Formula I, its maleate salt crystalline form 1 and its methanesulfonate salt crystalline form 1

Samples and preparation of experimental: An appropriate amount of maleate salt crystalline form 1, methanesulfonate salt crystalline form 1 and the compound of Formula I were put in watch glasses, and then spread into a thin layer about 3-5 mm in thickness.

Experimental conditions: Under conventional(25 °C, avoid light), high temperature(60°C, avoid light), high temperature and high humidity(40°C/75%RH, open and avoid light), light(25°C,45001ux ± 500lux, open)and oxidation(40°C, container closure containing urea hydrogen peroxide, open and avoid light) conditions for 14 days.

Test items: Sampled on 0^{th} day and 14^{th} day, the XRD, TGA and HPLC were tested and compared, the test results on the 14^{th} day were show in Table 27.

**Table 27 Stability test results on the 14^{th} day**

| items | Crystalline form | conventional | high temperature | high temperature and high humidity | light | oxidation |
|---|---|---|---|---|---|---|
| Crystalline form | compound of Formula I | unchanged | transformed | decrease crystallinity | transfermed | transformed |
| | mesylate salt crystalline Form 1 | unchanged | unchanged | unchanged | unchanged | decrease crystallinity |
| | maleate salt crystalline Form 1 | unchanged | unchanged | unchanged | unchanged | unchanged |
| Weight loss | compound of Formula I | 20%-22% | about 18% | 20%-22% | 20%-22 % | about 27% |
| | mesylate salt crystalline Form 1 | 5%-6% | 5%-6% | 5%-6% | 5%-6% | >8% |
| | maleate salt crystalline Form 1 | <1% | <1% | <1% | <1% | <1% |
| Decrease value of the main ingredient content | compound of Formula I | <0.4% | 13.5% | <0.4% | 5.7% | 40.3% |
| | mesylate salt crystalline Form 1 | <0.3% | <0.3% | <0.3% | 0.6% | 86.8% |
| | maleate salt crystalline Form 1 | <0.1% | <0.1% | <0.1% | 0.7% | 5.3% |

According to the result data:
(1) XRD detection showed that maleate salt crystalline form 1 had no significant change after 14 days under conventional, high temperature, high temperature and high humidity, light and oxidation conditions. The crystallinity of mesylate salt crystalline form 1 was decreased after 14 days under oxidizing conditions. The stability of crystalline form of the compound of Formula I was relatively low, and the specific XRD spectra were shown in Figure 46-48.
(2) TGA test showed that maleate salt crystalline form 1 had no significant change in weight loss after 14 days under conventional, high temperature, high temperature and high humidity, light and oxidation conditions, all the weight lossbefore 120°C were less than 1%. The weight loss of mesylate salt crystalline form 1 was slightly increased after 14 days under oxidizing conditions. The specific TGA charts were shown in Figure 49-50.
(3) HPLC test results showed that the purity decrease of the main ingredient content of maleate salt crystalline form 1 was less than 0.1% after 14 days under conventional, high temperature, high temperature and high humidity conditions; the purity decrease of the main ingredient content was approximately 0.7% after 14 days under light condition; the purity decrease of the main ingredient content was about 5.3% after 14 days under oxidation condition; while the purity of the main ingredient content of the compound of Formula I and mesylate salt crystalline form 1 were significantly decreased under light condition.

### Example 20: Stability assessment of maleate salt crystalline form 2 of the compound of Formula I

After maleate salt crystalline form 2 was placed at room temperature for 1 day or heated to 120°C, the stability of crystalline form was tested by XRD, the specific test results were shown in Figure 51 and Figure 52.

According to the test results, the crystallinity of maleate salt crystalline form 2 was significantly worse after 1 day at room temperature; after the temperature was raised to 120°C, the crystalline state of the maleate salt crystalline form 2 has changed, and part of it has transformed into maleate salt crystalline form 1.

### Example 21: Pharmacological test of the compound of Formula I

### Example A: Kinase assay

Method: The inhibitory activity of the compound of Formula I against FGFR1, FGFR2, FGFR3, FGFR4 and KDR was tested by mobility shift assay (concentration of ATP is Km value).

### Test method:

Reagent: basic kinase buffer: 50mM HEPES (pH 7.5); 0.0015% Brij-35
Stop buffer: 100mM HEPES(pH 7.5); 0.0015% Brij-35; 0.2% Coating Reagent #3; 50mM EDTA
Prepare compounds: dilute test compounds to specific concentration using 100% DMSO
Reaction process:
   1) prepare 2.5X enzyme solution
      add kinase to IX basic kinase buffer
   2) prepare 2.5X peptide solution
      add FAM-labeled peptide and ATP to IX basic kinase buffer FAM-labeled
   3) prepare analysis board
      transferr 10µL of test compound to 384-well plate, adding 90µL of 1X basic kinase buffer
   4) add 10µL of 2.5X enzyme solution to each well of the assay plate and incubating for 10min at room temperature
   5) add 10µL of 2.5X enzyme solution to each well of the assay plate and incubating for specific time at 28°C
   6) stop the reaction by adding 25 µL of stop solution to each well
   7) read data with Caliper and calculating IC50 value

**Table 28**

| IC₅₀ of compound of Formula I(nM) | | | | |
|---|---|---|---|---|
| FGFR1 | FGFR2 | FGFR3 | FGFR4 | KDR |
| 0.8 | 1.7 | 1.2 | 21.0 | 2.9 |

### Example B: Cell proliferation assay

Method 1: the growth inhibitory effect of the compound of Formula I on human tumor cell NCI-H1581 cultured in vitro was observed using CellTiter 96® AQᵤₑₒᵤₛ One Solution cell proliferation assay kit method.
Test method: 180µl of the cell suspension was added to a 96-well plate and placed in a CO₂ incubator overnight. Tested compounds were dissolved in DMSO and subjected to a 3-fold gradient dilution for a total of 10 concentrations. 20µl of the medium containing the test compound and DMSO was transferred to the corresponding cell wells respectively. Incubated in 5% CO₂ at 37°C for 144hrs. 40µl of CellTiter 96® AQueous One Solution cell proliferation assay reagent was added to detection board and was incubated in 5% CO₂ at 37°C for 2hrs. The IC₅₀ value was calculated by recording the light absorption value (OD490) at 490nm using VICTOR TM X5 instrument.
Method 2: the growth inhibitory effect of the compound of Formula I on human tumor cell NCI-H1581 and SNU-16 cultured in vitro was observed using CellTiter Glo assay method.
Test method: the cells were added appropriate volume of whole medium to suspend. 100µl of the cell suspension was added to a 96-well plate and placed in a CO₂ incubator overnight. Tested compounds were dissolved in DMSO and subjected to a 3-fold gradient dilution for a total of 10 concentrations. Tested compound and DMSO reference materials were transferred to the corresponding wells containing 100µl of medium respectively. Incubated in 5% CO₂ at 37°C for 96hrs. 100µl of CellTiter-Glo reagent was added to the assay plate and incubated for 10min at room temperature to stabilize the luminescence signal. The IC₅₀ value was calculated by recording the RLU (relative luminescence unit) using VICTOR TM X5 instrument.

The test results showed that the IC₅₀ values of the compound of Formula I on NCI-H1581 cells and SNU-16 cells were 2.0nM and <1nM respectively.

### Example C: Xenograft tumor model test 1

Reagent: DMSO, polyethylene glycol-15-hydroxystearate (Solutol), saline.
Animals: BALB/C-nude strains nude mice: SPF animals, weighing 18∼22g, female, provided by Vitrallihua Experimental Animal Technology Co., Ltd. Fed with SPF feed, freely drink distilled water.
Human cancer cell line: NCI-H1581 human non-small cell lung cancer cell line, provided by Shanghai Ruizhi Chemical Research Co., Ltd..
Method of tumor transplantation assay: The NCI-H1581 cell line was inoculated subcutaneously into the right axilla of BALB/C nude mice with an amount of 1x10⁷/100µL/cell in an ultra-clean bench under sterile operation. After 10 days, the tumors were grown and touched (about 100-200 mm³). The animals were randomly divided into groups of 6 animals and each was weighted and labled. The experimental group was intragastrically administered the compound of Formula I once a day. Nude mice were kept in a room with a temperature of 20-22 °C, relative humidity of 40-60%, and the shielding system was supplemented by the environment of a clean laminar flow cabinet. The subcutaneous tumor volume was measured with a caliper every 3-4 days since the experiment started, and the tumor growth curve was drawn to calculate the tumor inhibition rate. The test results were statistically analyzed using GraphPad Prism 5 software, and the experimental data are shown in Figures 53.

### Example D: Xenograft tumor model test 2

Reagent: MSO, polyethylene glycol-15-hydroxystearate (Solutol), saline.
Animals: BALB/C-nude strains nude mice: SPF animals, weighing 18∼22g, female, provided by Vitrallihua Experimental Animal Technology Co., Ltd. Fed with SPF feed, freely drink distilled water.
Human cancer cell line: SNU-16 human non-small cell lung cancer cell line, provided by ATCC, batch number was CRL-5974™.
Method of tumor transplantation assay: The SNU-16 cell line was inoculated into the right back of BALB/C nude mice with an amount of 0.2mL(3×10⁶ pcs+Matrigel)/one mouse in an ultra-clean bench under sterile operation, and when the average tumor volume reached 151mm³, the drug was administered in groups. The animals were randomly divided into groups of 6 animals, each was weighted and labeled. The positive group was intragastrically administered JNJ42756493 once a day. The experimental group was intragastrically administered the compound of Formula I once a day. Nude mice were kept in a room temperature of 20-22°C, relative humidity of 40-60%, and the shielding system was supplemented by the environment of a clean laminar flow cabinet. The subcutaneous tumor volume was measured with a caliper twice a week after the experiment, and the tumor growth curve was drawn to calculate the tumor inhibition rate. The test results were statistically analyzed using GraphPad Prism 5 software, and the experimental data are shown in Figures 54.

### Example 22: Pharmacokinetic study of the compound of Formula I and maleate salt crystalline Form 1 thereof

Drugs and reagents: 6-(2-chloro-3,5-dimethoxyphenyl)-N-(4-(4-morpholinopiperidin-1-yl)phenyl)-[1,2,4]t riazolo[4',3':1,6]pyrido[2,3-d]pyrimidin-2-amine(compound of Formula I), and maleate salt crystalline form 1 of the compound of Formula I used in this study were grounded to fine particles. The material content (purity) was not less than 99.0%.
Experimental animals: SD rats were randomly divided into the compound of Formula I group and maleate salt crystalline form 1 gurop, each group including three male rats.
Pharmaceutical preparation: each compound was prepared into a clear solution in 10%DMSO/10%Solutol/80%Water solution, the final concentration of each compound was 1mg/mL.
Administration and sample collection: each suspension was orally administered to fasted SD rats with a dose volume of 10 mL/kg and an administration dose of 10 mg/kg. The blood samples were collected in EDTA-K pre-anticoagulantion tubes before administration (0h) and at 0.25h, 0.5h, 1h, 2h, 4h, 7h and 24h after administration. The plasma was separated from the samples by centrifugation at 4000 rpm for 10 minutes at 4°C. The plasma was collected and stored at -80°C for analysis.

The samples were analyzed by AB4000 API LC/MS combined with HPLC. Under liquid chromatography conditions, a phenomenex C18 2.6u(50×2.1mm)column was used as the stationary phase, acetonitrile-water and 0.1% formic acid were used as the mobile phase, and the injection volume was 10 µL. The PK data of the compound of Formula I and its maleate salt crystalline Form 1 were shown in Table 29 and Figure 55. The absorption value of the maleate salt crystalline Form 1 of the compound of Formula I was higher than that of the compound of Formula I.

**Table 29**

| | AUC₍₀₋₂₄₎ (ng/mL^{∗}h) | AUC_{(0-∞)} (ng/mL^{∗}h) | T_{1/2} (h) | Tₘₐₓ (h) | Cₘₐₓ (ng/mL) |
|---|---|---|---|---|---|
| compound of Formula I | 5871 | 6523 | 7.9 | 6 | 443 |
| maleate salt crystalline Form 1 of the compound of Formula I | 6862 | 7137 | 5.1 | 4 | 509 |

### Example 23: Pharmacokinetic study of the compound of Formula I and salt forms thereof

Drugs and reagents: 6-(2-chloro-3,5-dimethoxyphenyl)-N-(4-(4-morpholinopiperidin-1-yl)phenyl)-[1,2,4]t riazolo[4',3':1,6]pyrido[2,3-d]pyrimidin-2-amine(compound of Formula I) and salt forms thereof which include malate salt crystalline form, tartrate salt crystalline form, phosphate salt crystalline form, methanesulfonate salt crystalline form, hydrochloride salt crystalline form, fumarate salt crystalline form, citrate salt crystalline form, besylate salt crystalline form used in this study were ground to fine particles. The material content (purity) was not less than 99.0%.

Experimental animals: SD rats were randomly divided into the compound of Formula I group and each salt crystalline form of the compound of Formula I gurop, each group including three male rats.

Pharmaceutical preparation: each compound was prepared in purified water, wherein, the mesylate salt crystalline form, hydrochloride salt crystalline form and besylate salt crystalline form were prepared into a clear solution, the malate salt crystalline form, tartrate salt crystalline form, phosphate salt crystalline form, fumarate salt crystalline form, citrate salt crystalline form and sulfate salt crystalline form were prepared into a suspension, and final concentration of each compound was 0.5mg/mL.

Administration and sample collection: each suspension was orally administered to fasted SD rats with a dose volume of 10 mL/kg and an administration dose of 10 mg/kg. The blood samples were collected in EDTA-K pre-anticoagulantion tubes before administration (0h) and at 0.25h, 0.5h, 1h, 2h, 4h, 7h and 24h after administration. The plasma was separated from the samples by centrifugation at 4000 rpm for 10 minutes at 4°C. The plasma was collected and stored at -80°C for analysis.

The samples were analyzed by AB4000 API LC/MS combined with HPLC. Under liquid chromatography conditions, a phenomenex C18 2.6u(50×2.1mm)column was used as the stationary phase, acetonitrile-water and 0.1% formic acid were used as the mobile phase, and the injection volume was 10 µL. The PK data of the compound of Formula I and its salt crystalline Form were shown in Table 30. The absorption value of the tartrate salt crystalline form of the compound of Formula I was higher than that of other compound.

**Table 30**

| | FB¹ | M² | T³ | P⁴ | Ms⁵ | HCl⁶ | FA⁷ | C⁸ | BA⁹ |
|---|---|---|---|---|---|---|---|---|---|
| Cₘₐₓ (ng/mL) | 179 | 210 | 290 | 230 | 190 | 212 | 252 | 130 | 200 |
| AUC₍₀₋₂₄₎ (ng/mL^{∗}h) | 786 | 971 | 1360 | 925 | 883 | 981 | 1059 | 487 | 879 |

| | | | | | | | | | |
|---|---|---|---|---|---|---|---|---|---|
| ¹ the compound of Formula I ² L- malate salt crystalline form 1 of the compound of Formula I ³ L-tartrate salt crystalline form 1 of the compound of Formula I ⁴ phosphate salt crystalline form 1 of the compound of Formula I ⁵ methanesulfonate salt crystalline form 1 of the compound of Formula I ⁶ hydrochloride salt crystalline form 1 of the compound of Formula I ⁷ fumarate salt crystalline form 1 of the compound of Formula I ⁸ citrate salt crystalline form 1 of the compound of Formula I ⁹ besylate salt crystalline form 1 of the compound of Formula I | | | | | | | | | |

### Example 24: The first capsule preparation method

The formulationwas shown in the following table:

**Table 31**

| Ingredient | 5mg Hard capsule | 2mg Hard capsule | 0.5mg Hard capsule |
|---|---|---|---|
| Batch(number of capsules) | 20000 | 20000 capsules | 10000 capsules |
| API ² | 138.60¹ | 55.44¹ | 6.93¹ |
| Mannitol | 2681.40 | 1824.56 | 745.07 |
| Crospovidone | 150.00 | 100.00 | 40.00 |
| Magnesium stearate | 30.00 | 20.00 | 8.00 |
| Total weight | 3000.00 | 2000.00 | 800.00 |

| | | | |
|---|---|---|---|
| ¹ The unit content of the active ingredient has been adjusted according to the salinity coefficient ² The API described here specifically refers to maleate salt crystalline form 1 of the compound of Formula I. | | | |

The preparation process of the capsulewas described as follows:
1) API sieving
   Sieved API manually or using a crushing equipment equipped with a suitable sieve mesh size before added,.
2) Blending
   Weighed the sieved API, mannitol and crospovidone, and then transferred them to a square cone mixer for blending.
3) Final blending
   Magnesium stearate was weighed and added into the square cone mixer to continue blending.
4) Capsule filling
   The 5mg size was filled with No. 2 gelatin capsule shell, the 2mg size was filled with No. 3 gelatin capsule shell, and the 0.5mg size was filled with No. 4 gelatin capsule shell.
5) Package
   This product was packaged in oral solid drug pharmaceutically acceptable high-density polyethylene bottle and oral solid drug pharmaceutically acceptable polypropylene/low-density polyethylene moisture-proof combination cover, the packaged product was stored at room temperature.

### Example 25: The second capsule preparation method

The formulation was shown in the following table:

**Table 32**

| Ingredient | 5mg Hard capsule | 2mg Hard capsule | 0.5mg Hard capsule |
|---|---|---|---|
| Batch(number of capsules) | 20000 capsules | 20000 capsules | 10000 capsules |
| API² | 138.60¹ | 55.44¹ | 6.93¹ |
| Microcrystalline cellulose | 1481.40 | 1024.56 | 425.07 |
| Spray dried lactose | 1200.00 | 800.00 | 320.00 |
| Crospovidone | 150.00 | 100.00 | 40.00 |
| Magnesium stearate | 30.00 | 20.00 | 8.00 |
| Total weight | 3000.00 | 2000.00 | 800.00 |

| | | | |
|---|---|---|---|
| ¹ The unit content of the active ingredient has been adjusted according to the salinity coefficient ² The API described here specifically refers to maleate salt crystalline form 1 of the compound of Formula I. | | | |

The preparation process of the capsule was as follows:
1) API sieving
   Sieved API manually or using a crushing equipment equipped with a suitable sieve mesh size before added,.
2) Blending
   Weighed the sieved API, microcrystalline cellulose, spray dried lactose and crospovidone, and then transferred them to a square cone mixer for blending.
3) Final blending
   Magnesium stearate was weighed and added into the square cone mixer to continue blending.
4) Capsule filling
   The 5mg size was filled with No. 2 gelatin capsule shell, the 2mg size was filled with No. 3 gelatin capsule shell, and the 0.5mg size was filled with No. 4 gelatin capsule shell.
5) Package
   This product was packaged in oral solid drug pharmaceutically acceptable high-density polyethylene bottle and oral solid drug pharmaceutically acceptable polypropylene/low-density polyethylene moisture-proof combination cover, the packaged product was stored at room temperature.

The exemplary embodiments of the present invention have been described above. However, the technical solution of the present invention should not be limited to this. Those skilled in the art should understand that any modification, equivalent replacement, improvement, etc. made within the spirit and principle scope of the present invention should fall within the scope of protection of the present invention.

## Claims

1. A salt or crystalline form of the compound of Formula I:

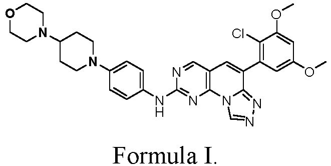

2. The salt or crystalline form according to claim 1, wherein the salt is selected from maleate, mesylate, besylate, hydrochloride, phosphate, L-tartrate, L-malate, citrate and fumarate salts.

3. The salt or crystalline form according to claim 1 or 2, wherein the salt is maleate salt, and the maleate salt of the compound of Formula I is crystalline form.

4. The salt or crystalline form according to claim 3, wherein the X- ray powder diffraction pattern of said crystalline form of the maleate salt comprises characteristic peaks with diffraction angles 2θ of 5.6°±0.2°, 8.4°±0.2°, 11.2°±0.2°, 22.6°±0.2° and 28.3°±0.2°.

5. The salt or crystalline form according to claim 3, wherein the X- ray powder diffraction pattern of said crystalline form of the maleate salt comprises characteristic peaks with diffraction angles 2θ of 5.6°±0.2°, 8.4°±0.2°, 11.2°±0.2°, 17.0 °±0.2°, 19.7°±0.2°, 22.6°±0.2°, 25.4°±0.2° and 28.3°±0.2°.

6. The salt or crystalline form according to claim 3, wherein the X- ray powder diffraction pattern of said crystalline form of the maleate salt comprisescharacteristic peaks with diffraction angles 2θ of 5.6°±0.2°, 8.4°±0.2°, 11.2°±0.2°, 17.0 °±0.2°, 19.7°±0.2°, 22.6°±0.2°, 23.3 °±0.2°, 25.4°±0.2° and 28.3°±0.2°.

7. The salt or crystalline form according to claim 3, wherein the X- ray powder diffraction pattern of said crystalline form of the maleate salt comprises characteristic peaks with diffraction angles 2θ of 5.6°±0.2°, 8.4°±0.2°, 11.2°±0.2°, 14.0°±0.2°, 17.0 °±0.2°, 19.7°±0.2°, 22.6°±0.2°, 23.3 °±0.2°, 25.4°±0.2° and 28.3°±0.2°.

8. The salt or crystalline form according to claim 3, wherein the X- ray powder diffraction pattern of said crystalline form of the maleate salt is approximately as shown in Figure 1.

9. The salt or crystalline form according to claim 3, wherein the X- ray powder diffraction pattern of said crystalline form of the maleate salt is approximately as shown in Figure 2.

10. The salt or crystalline form according to claim 1 or 2, wherein the salt is mesylate salt, and the mesylate salt of compound of Formula I is crystalline form, which has an X- ray powder diffraction pattern comprising characteristic peaks with diffraction angles 2θ of 4.7°±0.2°, 9.4°±0.2° and 14.1°±0.2°.

11. The salt or crystalline form according to claim 10, wherein the X-ray powder diffraction pattern of said crystalline form of the mesylate salt comprises characteristic peaks with diffraction angles 2θ of 4.7°±0.2°, 9.4°±0.2°, 10.7°±0.2°, 12.1°±0.2°, 14.1°±0.2° and 19.0°±0.2°.

12. The salt or crystalline form according to claim 10, wherein the X- ray powder diffraction pattern of said crystalline form of the mesylate salt comprises characteristic peaks with diffraction angles 2θ of 4.7°±0.2°, 9.4°±0.2°, 10.7°±0.2°, 12.1°±0.2°, 14.1°±0.2°, 16.3°±0.2°, 16.8°±0.2° and 19.0°±0.2°.

13. The salt or crystalline form according to claim 10, wherein the X- ray powder diffraction pattern of said crystalline form of the mesylate salt is approximately as shown in Figure 12.

14. A pharmaceutical composition comprising a therapeutically effective amount of the salt or crystalline form according to any one of claims 1-13, and a pharmaceutically acceptable excipient, adjuvant and/or carrier.

15. The pharmaceutical composition according to claim 14, wherein said pharmaceutical composition is administrated orally.

16. Use of the salt and/or crystalline form according to any one of claims 1-13 or the pharmaceutically composition according to claim 14 or 15 in the manufacture of a medicament, wherein the medicament is used in treating, preventing, delaying or arresting the occurrence or progression in cancer or cancer metastasis.

17. Use according to claim 16, wherein the compound is used in the manufacture of a medicament for treating a disease mediated by FGFR.

18. Use according to claim 17, wherein the FGFR comprises FGFR1, FGFR2, FGFR3 or FGFR4.

19. Use according to claim 16, wherein the cancer is selected from the group consisting of breast cancer, multiple myeloma, bladder cancer, endometrial cancer, stomach cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumor, intracranial tumor, hepatobiliary duct cell carcinoma, myelodysplastic syndrome, malignant glioma, prostate cancer, thyroid cancer, Schwann cell tumor, lung squamous cell carcinoma, lichenoid keratosis, synovial sarcoma, skin cancer, pancreatic cancer, testicular cancer and liposarcoma.

20. A method of treating or preventing a disease mediated by FGFR, wherein a therapeutically effective amount of the salt and/or crystalline form according to any one of claims 1-13, or the pharmaceutical composition according to claim 14 or 15 is administered to a treatment subject.

21. The method according to claim 20, wherein the FGFR comprises FGFR1, FGFR2, FGFR3 or FGFR4.

22. The method according to claim 21, wherein the disease mediated by FGFR is cancer.

23. The method according to claim 23, wherein the cancer is selected from the group consisting of breast cancer, multiple myeloma, bladder cancer, endometrial cancer, stomach cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumors, intracranial tumor, hepatobiliary duct cell carcinoma, myelodysplastic syndrome, malignant glioma, prostate cancer, thyroid cancer, Schwann cell tumor, lung squamous cell carcinoma, lichenoid keratosis, synovial sarcoma, skin cancer, pancreatic cancer, testicular cancer and liposarcoma.

24. The method according to any one of claims 20-23, wherein the treatment subject is human.
